# FASCICULE DE BREVET EUROPEEN

(11) **EP 2 718 462 B1**
(45) Date de publication et mention de la délivrance du brevet: **07.03.2018**
(21) Numéro de dépôt: 12730411.1
(22) Date de dépôt: 07.06.2012
(51) Int. Cl.: C12Q 1/68, G01N 33/574, C12N 15/11

(54) **METHODE DE PREDICTION DE LA REPONSE A UN TRAITEMENT AVEC UN AGENT BLOQUANT HER2**
VERFAHREN ZUR VORHERSAGE DER REAKTION AUF EINE BEHANDLUNG MIT EINEM HER2-BLOCKER
METHOD FOR PREDICTING THE RESPONSE TO TREATMENT WITH AN HER2-BLOCKING AGENT

(30) Priorité: 10.06.2011 FR 1155128
(43) Date de publication de la demande: 16.04.2014
(73) Titulaire: Université de Nice Sophia Antipolis, 06000 Nice (FR); Centre National de la Recherche Scientifique (C.N.R.S.), 75016 Paris (FR)
(72) Inventeur: PAGES, Gilles, MC-98000 Monaco (MC)
(74) Mandataire: Regimbeau
(86) Numéro de dépôt international: PCT/EP2012/060807
(87) Numéro de publication internationale: WO 2012/168370

(56) Documents cités:
- WO-A1-2006/010938
- WO-A1-2006/098978
- WO-A1-2011/071232
- WO-A2-01/79226
- US-A1- 2010 055 705
- CARRICK D M ET AL: "Genetic variations in ZFP36 and their possible relationship to autoimmune diseases", JOURNAL OF AUTOIMMUNITY, LONDON, GB, vol. 26, no. 3, 1 mai 2006 (2006-05-01), pages 182-196, XP024910079, ISSN: 0896-8411, DOI: 10.1016/J.JAUT.2006.01.004 [extrait le 2006-05-01]
- S. E. BRENNAN ET AL: "The mRNA-Destabilizing Protein Tristetraprolin Is Suppressed in Many Cancers, Altering Tumorigenic Phenotypes and Patient Prognosis", CANCER RESEARCH, vol. 69, no. 12, 2 juin 2009 (2009-06-02), pages 5168-5176, XP055018341, ISSN: 0008-5472, DOI: 10.1158/0008-5472.CAN-08-4238 cité dans la demande
- E. SUSWAM ET AL: "Tristetraprolin Down-regulates Interleukin-8 and Vascular Endothelial Growth Factor in Malignant Glioma Cells", CANCER RESEARCH, vol. 68, no. 3, 1 février 2008 (2008-02-01), pages 674-682, XP055018342, ISSN: 0008-5472, DOI: 10.1158/0008-5472.CAN-07-2751 cité dans la demande
- N AL-SOUHIBANI ET AL: "The RNA-binding zinc-finger protein tristetraprolin regulates AU-rich mRNAs involved in breast cancer-related processes", ONCOGENE, vol. 29, no. 29, 24 mai 2010 (2010-05-24), pages 4205-4215, XP055018343, ISSN: 0950-9232, DOI: 10.1038/onc.2010.168 cité dans la demande
- ELIZABETH VAN TUBERGEN ET AL: "Tristetraprolin regulates interleukin-6, which is correlated with tumor progression in patients with head and neck squamous cell carcinoma", CANCER, vol. 117, no. 12, 10 janvier 2011 (2011-01-10), pages 2677-2689, XP055018131, ISSN: 0008-543X, DOI: 10.1002/cncr.25859
- HYUN HEE LEE ET AL: "Tristetraprolin regulates expression of VEGF and tumorigenesis in human colon cancer", INTERNATIONAL JOURNAL OF CANCER, vol. 126, 1 janvier 2009 (2009-01-01), pages 1817-1827, XP055018146, ISSN: 0020-7136, DOI: 10.1002/ijc.24847
- P. GRISERI ET AL: "A synonymous polymorphism of the Tristetraprolin (TTP) gene, an AU-rich mRNA-binding protein, affects translation efficiency and response to Herceptin treatment in breast cancer patients", HUMAN MOLECULAR GENETICS, vol. 20, no. 23, 29 août 2011 (2011-08-29) , pages 4556-4568, XP055018206, ISSN: 0964-6906, DOI: 10.1093/hmg/ddr390

## Description

La présente invention se rapporte au domaine de la prédiction de la réponse d'un sujet à un traitement anti-cancéreux. En particulier, elle concerne une méthode de prédiction de la réponse d'un sujet à un traitement avec un agent bloquant HER2.

Le récepteur membranaire HER2 (« Human Epidermal Growth Factor Receptor-2 ») appartient à la famille du récepteur au facteur de croissance épidermique (EGFR). L'oncoprotéine HER2 de 185 kDa consiste en un domaine extracellulaire, un domaine transmembranaire et une région intracellulaire ayant une activité tyrosine kinase intrinsèque (Bargman *et al.,* 1986; Yamamoto *et al.* 1986). La glycoprotéine transmembranaire HER2 possède ainsi une activité tyrosine kinase conduisant à une activation de la transcription de gènes régulant la progression du cycle cellulaire.

La surexpression de la protéine HER2 a été identifiée dans différents cancers, nommés « cancers HER2-positifs », incluant notamment les cancers du sein (principale cause de décès de femmes atteintes de cancer dans le monde), de l'ovaire, du côlon, du pancréas, de la prostate et de l'estomac. Cette surexpression est corrélée à une plus grande agressivité tumorale, un risque accru de récidive et à un mauvais pronostic. En particulier, des études ont montré que le gène codant la protéine HER2 était amplifié de 2 à plus de 20 fois dans 30% des cas de cancers du sein invasifs et que cette amplification est associée à un très mauvais pronostic vital des patientes (Slamon *et al.,* 1987).

Cependant, le développement d'anticorps thérapeutiques dirigés contre HER2, en particulier l'anticorps monoclonal trastuzumab (commercialisé notamment sous le nom Herceptin® F. Hoffmann-La Roche Ltd, Basel, Switzerland et Genentech, Inc., South San Francisco, CA) a permis de modifier ce pronostic (De Laurentiis *et al.,* 2005). Ainsi, le traitement avec le trastuzumab de patientes atteintes d'un cancer du sein HER2-positif a permis d'augmenter de manière très significative le taux de survie global de ces patientes (Gianni *et al.,* 2011).

Bien que le trastuzumab constitue un remarquable progrès dans le traitement du cancer du sein HER2-positif, cet anticorps n'est malheureusement pas efficace sur l'ensemble des patientes. En effet, certains malades sont réfractaires ou développent une résistance au traitement avec le trastuzumab, généralement dans l'année suivant son initiation en situation métastatique (Nahta *et al.,* 2006).

En outre, ce type de traitement présente l'inconvénient d'être très onéreux.

Il est donc intéressant de disposer d'une méthode fiable permettant de prédire la réponse d'un sujet à un traitement avec un agent bloquant HER2, en particulier le trastuzumab ; une telle méthode permettant d'adapter le traitement thérapeutique à chaque patient, d'éviter des éventuels effets secondaires, de développer des thérapies alternatives et de réduire les dépenses de santé.

On connait dans l'art antérieur, des méthodes visant à prédire la réponse d'un sujet à un traitement avec un agent bloquant HER2, en particulier le trastuzumab, notamment basées sur la détermination de l'expression de plusieurs gènes.

Ainsi, la Demande Internationale WO 2009/150127 enseigne une méthode de prédiction de la réponse d'un patient à un traitement avec un agent bloquant HER2, comprenant la détermination de l'expression d'au moins 4 gènes. En particulier, les inventeurs de cette Demande ont identifié un profil d'expression de 28 gènes permettant de prédire la réponse d'une patiente atteinte d'un cancer du sein HER2-positif à un traitement avec le trastuzumab (Végran *et al.,* 2009). Toutefois, une telle méthode présente certains inconvénients. En effet, elle nécessite l'analyse de l'expression d'un nombre élevé de gènes qui peut être longue et coûteuse. En outre, elle requiert le prélèvement d'un échantillon de la tumeur chez le patient, qui peut nécessiter une opération chirurgicale, risquer de favoriser le développement de cellules malignes des tissus voisins, laisser des séquelles (cicatrice...) ou encore être douloureux.

Des méthodes de prédiction de la réponse à un traitement ERBB2/HER2 et de prédiction de la réponse d'un patient à un traitement qui inhibe la capacité de signal de la protéine HER2 sont décrites dans les demandes internationales WO 2006/010938 et WO 2006/098978 respectivement. Toutefois, même si ces méthodes donnent des résultats satisfaisants, leur mise en oeuvre n'est pas aisée et leur précision doit être améliorée.

Certaines méthodes sont quant à elles basées sur la détection du taux de la protéine HER2 dans les cellules cancéreuses circulantes, telles qu'illustrées dans la Demande Internationale WO 2006/041959. Toutefois, ces méthodes peuvent être difficiles à mettre en oeuvre et requièrent un équipement particulier onéreux qui n'est à l'heure actuelle pas présent dans tous les centres médicaux.

Ainsi, les méthodes connues dans l'art antérieur peuvent notamment présenter les inconvénients d'être difficiles à mettre en oeuvre, onéreuses et/ou peu fiables.

Il subsiste donc un besoin pour des méthodes de prédiction de la réponse d'un sujet à un traitement avec un agent bloquant HER2, présentant des caractéristiques et conditions d'applications améliorées, notamment en terme économique, de simplicité, de rapidité et/ou de fiabilité, qui soient utilisables dans le plus grand nombre de centres médicaux ; ces méthodes permettant notamment aux cliniciens de prendre la décision thérapeutique la plus adaptée pour chaque patient souffrant de pathologie liée à HER2, en particulier de cancer HER2-positif, notamment de cancer du sein HER2-positif.

De manière surprenante les inventeurs ont maintenant identifié un polymorphisme silencieux dans le gène codant la protéine tristétraproline (TTP) dont la présence est corrélée à une réduction de la traduction de cette protéine et à une absence de réponse au traitement avec un agent bloquant HER2.

Cette corrélation est particulièrement inattendue. En effet, des études ont montré que la protéine tristétraproline permet de réguler négativement l'expression des ARNm de différents gènes surexprimés dans divers cancers, en diminuant leur temps de demi-vie. Ces ARNm appartiennent à la famille des ARE-ARNm (ARNm contenant des éléments riches en AU dans la région 3' non traduite ou « 3' untranslated région » ou 3'UTR) et sont les produits de gènes impliqués dans divers processus de contrôle cellulaire, tels que la division cellulaire, l'apoptose et l'angiogenèse. Une dérégulation de l'expression de ces ARE-ARNm, à savoir une surexpression de ces ARE-ARNm, conduit à un phénotype oncogénique.

Ainsi, dans le cas du cancer du sein, les ARNm cibles régulés par la protéine tristétraproline ont été identifiés comme étant des ARE-ARNm, produits de différents gènes dont uPA, MMPA et uPAR (Al-Souhibani et al., 2010).

Or, bien que la surexpression de HER2 ait été démontrée dans différents cancers (notamment le cancer du sein) et que HER2 soit impliquée dans la progression du cycle cellulaire, l'ARNm codant HER2 n'appartient pas à la famille des ARE-ARNm et est encore moins une cible de la protéine tristétraproline.

Ainsi, selon un premier aspect, l'invention a pour objet une méthode *in vitro* ou *ex vivo* de prédiction de la réponse d'un sujet à un traitement avec au moins un agent bloquant HER2, ladite méthode comprenant les étapes de :
i) identification du nucléotide au niveau du site polymorphe rs3746083 367C>T sur la séquence SEQ ID NO : 3, pour au moins un allèle (au moins une copie), en particulier les deux allèles (les deux copies) du gène codant la protéine tristétraproline, dans un échantillon biologique dudit sujet ; et/ou
ii) détermination du taux de protéine tristétraproline, dans un échantillon biologique dudit sujet,
dans laquelle ledit sujet est atteint d'un cancer du sein HER2-positif, et ledit agent bloquant est le trastuzumab.

La méthode de prédiction selon l'invention présente notamment les avantages suivants :
- Elle est simple et rapide. En particulier, l'étape i) présente l'avantage de ne pas requérir le prélèvement d'un échantillon de la tumeur chez le sujet et d'éviter ainsi les différents risques et problèmes liés à la réalisation d'une biopsie. En effet, elle peut être mise en oeuvre à partir d'un simple échantillon sanguin de 5 à 10 ml d'un sujet par des techniques de biologie moléculaire simples. Elle nécessite simplement une plateforme standard de biologie moléculaire déjà présente dans de nombreux centres médicaux. En outre, l'étape i) présente l'avantage de permettre l'obtention d'un résultat particulièrement simple à analyser. L'étape ii) quant à elle présente l'avantage de nécessiter uniquement l'analyse du taux d'une seule protéine ;
- Elle est fiable, reproductible et peu coûteuse.

La mise en oeuvre des étapes i) et ii) dans la méthode selon l'invention présente l'avantage d'optimiser la prédiction de la réponse d'un sujet à un traitement avec au moins un agent bloquant HER2, en particulier le trastuzumab.

Afin de permettre une meilleure compréhension de la présente invention, certaines définitions sont fournies. Sauf indications particulières, les autres termes techniques employés dans la présente Demande doivent être interprétés selon leur sens habituel.

On entend par « HER2 » au sens de la présente invention, l'oncoprotéine de 185 KDa, aussi nommée *erb*B-2*,* ERBB2 ou encore NEU. En particulier, HER2 a la séquence d'acides aminés SEQ ID N°1 (Référence NCBI: NP_004439.2).

On entend par « agent bloquant HER2 » au sens de la présente invention, toute molécule (telle que des molécules d'acides nucléiques incluant des molécules d'ADN, d'ARN tels que des molécules d'ARN interférents, des peptides, des protéines, des anticorps, des fragments d'anticorps...) inhibant significativement les fonctions de HER2, en particulier inhibant significativement l'activité tyrosine kinase de HER2 et/ou l'expression de HER2.

Une inhibition significative des fonctions de HER2, en particulier de l'activité tyrosine kinase de HER2 et/ou de l'expression de HER2 peut correspondre à une réduction d'au moins 10%, d'au moins 15%, d'au moins 20%, d'au moins 25%, d'au moins 30%, d'au moins 35% d'au moins 40%, d'au moins 45%, en particulier d'au moins 50% des fonctions de HER2, en particulier de l'activité tyrosine kinase de HER2 et/ou de l'expression de HER2 par rapport à un contrôle en l'absence d'un agent bloquant HER2.

Ledit agent bloquant HER2 peut se lier au domaine extracellulaire de HER2, inhiber l'homodimérisation et/ou l'héterodimérisation de HER2, se lier au domaine intracellulaire de HER2, inhiber le domaine tyrosine kinase de HER2 et/ou encore inhiber l'expression du gène codant HER2, tel que décrit dans l'article de Chen *et al.* (2003).

Selon la présente description, ledit agent bloquant HER2 peut être choisi dans le groupe consistant en :
- les anticorps dirigés contre le domaine extracellulaire de HER2, en particulier le trastuzumab (Herceptin®) ;
- les anticorps dirigés contre HER2 et inhibant l'homodimérisation et/ou l'hétérodimérisation de HER2, tel qu'avec HER3, en particulier l'anticorps monoclonal pertuzumab (encore nommé 2C4, omnitarg®) ;
- les vaccins anti-HER2 ;
- les inhibiteurs de l'activité tyrosine kinase de HER2, en particulier l'emodine (3-methyl-1,6,8-trihydroxyanthraquinone), le curcumin, l'OSI-774 (Tarceva®), le ZD-1839 (Iressa®) le CI-1033 et le lapatinib (Tykerb®, GSK572016, GW572016; GlaxoSmithKline, Research Triangle Park, NC, USA) ;
- les anticorps simples chaînes intracellulaires dirigés contre HER2, en particulier dirigés contre le domaine extracellulaire de HER2. Ce type d'anticorps permet d'éviter le transit de HER2 à travers le réticulum endoplasmique ;
- les inhibiteurs de transcription du gène codant HER2, en particulier le gène adénoviral E1A ; et
- les inhibiteurs de traduction de l'ARNm codant HER2, tels que des oligonucléotides antisens et des ribozymes ;
ces différents types d'agents bloquants HER2 étant notamment illustrés dans l'article de Chen *et al.* (2003).

Ledit agent bloquant HER2 peut être identifié selon des techniques bien connues de l'Homme du Métier. A titre d'exemple, ledit agent bloquant HER2 peut être identifié par un procédé comprenant :
- la mise en contact d'un agent à tester avec une cellule exprimant HER2 ;
- la culture de ladite cellule dans des conditions d'expression de HER2 ;
- la détermination des fonctions de HER2 et/ou du niveau d'expression de HER2 ;
- la comparaison des fonctions de HER2, en particulier l'activité tyrosine kinase de HER2 et/ou du niveau d'expression de HER2 en présence et en l'absence dudit agent à tester ;
la diminution significative des fonctions de HER2, en particulier de l'activité tyrosine kinase et/ou du niveau d'expression de HER2 en présence dudit agent à tester étant indicative de la présence d'un agent bloquant HER2.

En particulier, ledit agent bloquant HER2 est un anticorps dirigé contre HER2, en particulier dirigé contre le domaine extracellulaire de HER2 et/ou inhibant l'homodimérisation et/ou l'hétérodimérisation de HER2 et tout particulièrement choisi dans le groupe consistant en le trastuzumab (Herceptin®) et le pertuzumab (encore nommé 2C4, omnitarg®) et encore plus particulièrement dans la présente invention ledit agent bloquant HER2 est le trastuzumab.

Tel qu'utilisé dans la présente Demande, le terme "anticorps" englobe les anticorps monoclonaux et polyclonaux intacts, les anticorps multispécifiques (par exemple, des anticorps bispécifiques) formés d'au moins deux anticorps intacts, et des fragments d'anticorps (par exemple, Fab', F'(ab)2, Fv, des anticorps à chaîne unique) pour autant qu'ils présentent l'activité biologique désirée.

On entend par « anticorps dirigé contre une protéine » au sens de la présente invention, tout anticorps qui se lie spécifiquement à cette protéine.

Les anticorps sont dits « se lier spécifiquement » si: 1) ils présentent un seuil d'activité de liaison, et/ou 2) ils n'ont pas de réaction croisée significative avec des polypeptides connexes. L'Homme du Métier, par exemple, par analyse de Scatchard (1949) ou par résonance plasmonique de surface, peut facilement déterminer l'affinité de liaison d'un anticorps.

On entend par « protéine tristétraproline » au sens de la présente invention, la protéine encore nommée ZFP36 (« Zinc Finger Protein 36 ») ou TTP ou G0S24 ou GOS24 ou TIS11ou NUP475 ou RNF162A; appartenant à la famille des protéines de liaison aux ARE-ARNm (ARNm contenant des éléments riches en AU dans la région 3'UTR). En particulier la protéine tristétraproline a la séquence SEQ ID NO :2 (Références GenBank: AAA61240.1, NCBI : NP_003398.1).

Par « site polymorphe rs3746083 » au sens de la présente invention, on entend la position rs3746083 sur le génome humain à laquelle existe le polymorphisme d'un seul nucléotide (encore nommé SNP pour « Single Nucleotide Polymorphism »). Les nucléotides au niveau du site polymorphe rs3746083 peuvent être les nucléotides A, C, G ou T, le nucléotide ancestral étant un nucléotide C (Références NCBI : NM_003407.2: 367C>A ; NM_003407.2: 367C>G ; NM_003407.2: 367C>T).

En particulier, le nucléotide au niveau du site polymorphe rs3746083 correspond au nucléotide en position 367 de la séquence SEQ ID NO: 3 (Référence NCBI : NM_003407.2).

En particulier, le nucléotide au niveau du site polymorphe rs3746083 est le nucléotide en position 367 de la séquence SEQ ID NO : 3 (Référence NCBI : NM_003407.2).

On entend par « polymorphisme rs3746083 » au sens de la présente invention, le polymorphisme d'un seul nucléotide (SNP) localisé en position rs3746083 sur le génome humain. Les allèles du polymorphisme rs3746083 peuvent être les allèles A, C, G ou T, l'allèle ancestral étant l'allèle C (Références NCBI : NM_003407.2: 367C>A ; NM_003407.2: 367C>G ; NM_003407.2: 367C>T).Dans la présente invention, le polymorphisme rs3746083 correspond au polymorphisme 367C>T sur la séquence SEQ ID NO : 3 (Référence NCBI : NM_003407.2).

L'étape i) d'identification du nucléotide au niveau du site polymorphe rs3746083, pour au moins un allèle (au moins une copie), en particulier les deux allèles (les deux copies) du gène codant la protéine tristétraproline, dans un échantillon biologique dudit sujet peut être mise en oeuvre par toute technique bien connue de l'Homme du Métier telle que par digestion enzymatique, séquençage, hybridation spécifique et/ou amplification spécifique.

Le séquençage peut être effectué en utilisant des techniques bien connues, notamment à l'aide de séquenceurs automatiques sur de l'ADN génomique, de l'ADNc ou de l'ARN du sujet comprenant le nucléotide au niveau du site polymorphe rs3746083.

L'amplification peut être réalisée selon diverses techniques connues, utilisant des amorces d'acides nucléiques spécifiques permettant d'amplifier de l'ADN génomique, de l'ADNc ou de l'ARN du sujet comprenant le nucléotide au niveau du site polymorphe rs3746083. En particulier, de telles amorces sont capables de s'hybrider spécifiquement avec des parties de l'ADN génomique, de l'ADNc ou de l'ARN du sujet qui flanquent le nucléotide au niveau du site polymorphe rs3746083.

A titre d'exemples de techniques d'amplification, on peut citer la réaction en chaîne par polymérase (PCR, « Polymerase Chain Reaction »), l'amplification par déplacement de brin (SDA, « Strand Displacement Amplification »), la réaction en chaîne par ligase (LCR, « Ligase Chain Reaction ») et l'amplification basée sur des séquences d'acides nucléiques (NASBA, « Nucleic Acid Sequence Based Amplification »). Ces techniques peuvent être réalisées en utilisant des réactifs et protocoles disponibles dans le commerce.

Les méthodes de détection par hybridation sont basées sur la formation d'hybrides spécifiques entre les séquences d'acides nucléiques complémentaires qui servent à détecter le polymorphisme et l'ADN génomique, l'ADNc ou l'ARN du sujet comprenant le nucléotide au niveau du site polymorphe rs3746083. En particulier, une technique de détection implique l'utilisation d'une sonde d'acides nucléiques spécifique de l'allèle C, de l'allèle T, de l'allèle A ou de l'allèle G du polymorphisme rs3746083, suivie de la détection de la présence d'un hybride. La sonde peut être en suspension ou immobilisée sur un substrat ou support, en particulier sur une puce. La sonde est généralement marquée de manière à faciliter la détection des hybrides ; les marqueurs pouvant être des marqueurs fluorescents, chimioluminescents, radioactifs, enzymatiques, des colorants, ou autres.

L'absence d'un nucléotide C au niveau du site polymorphe rs3746083 (l'absence de l'allèle C du polymorphisme rs3746083) peut être détectée aisément par digestion enzymatique avec l'enzyme de restriction HhaI, précédée d'une amplification de l'ADN génomique, de l'ADNc ou de l'ARN du sujet comprenant le nucléotide au niveau du site polymorphe rs3746083 et éventuellement suivie d'un séquençage des fragments amplifiés et digérés. En effet, l'absence d'un nucléotide C au niveau du site polymorphe rs3746083 (de l'allèle C du polymorphisme rs3746083) supprime le site de restriction de l'enzyme HhaI.

L'évaluation des résultats obtenus à l'étape i) permet de prédire la réponse du sujet à un traitement avec au moins un agent bloquant HER2, le trastuzumab.

Ainsi, dans la méthode de prédiction selon l'invention, l'absence de nucléotide C au niveau du site polymorphe rs3746083, pour au moins un allèle (au moins une copie), en particulier les deux allèles (les deux copies) du gène codant la protéine tristétraproline chez un sujet est indicative d'un risque de non-réponse dudit sujet à un traitement avec au moins un agent bloquant HER2, le trastuzumab. Ledit sujet est alors prédit comme non-répondeur audit traitement.

Ainsi, dans la méthode de prédiction selon l'invention, la présence d'un nucléotide T au niveau du site polymorphe rs3746083, pour au moins un allèle (au moins une copie), en particulier les deux allèles (les deux copies) du gène codant la protéine tristétraproline chez un sujet est indicative d'un risque de non-réponse dudit sujet à un traitement avec au moins un agent bloquant HER2, le trastuzumab. Ledit sujet est alors prédit comme non-répondeur audit traitement.

Ainsi, dans la méthode de prédiction selon l'invention, la présence d'un nucléotide T au niveau du site polymorphe rs3746083, pour au moins un allèle (au moins une copie), en particulier les deux allèles (les deux copies) du gène codant la protéine tristétraproline chez un sujet, est indicative d'un risque plus important de non-réponse dudit sujet à un traitement avec au moins un agent bloquant HER2, le trastuzumab, en comparaison avec un sujet exempt d'un nucléotide T au niveau du site polymorphe rs3746083, en particulier en comparaison avec un sujet dont les deux allèles (les deux copies) du gène codant la protéine tristétraproline contiennent un nucléotide C au niveau du site polymorphe rs3746083.

Ainsi, dans la méthode selon l'invention, l'identification d'une hétérozygotie (T/C) au niveau du site polymorphe rs3746083 chez un sujet (présence d'un nucléotide T au niveau du site polymorphe rs3746083, pour un allèle du gène codant la protéine tristétraproline et présence d'un nucléotide C au niveau du site polymorphe rs3746083, pour l'autre allèle du gène codant la protéine tristétraproline) est indicative d'un risque plus important de non-réponse dudit sujet à un traitement avec au moins un agent bloquant HER2, le trastuzumab, en comparaison avec un sujet présentant une homozygotie (C/C) au niveau du site polymorphe rs3746083.

Le sujet présentant une hétérozygotie (T/C) au niveau du site polymorphe rs3746083 est prédit comme non-répondeur à un traitement avec au moins un agent bloquant HER2, le trastuzumab.

Le sujet présentant une homozygotie (C/C) au niveau du site polymorphe rs3746083 est prédit comme répondeur à un traitement avec au moins un agent bloquant HER2, le trastuzumab.

Ainsi, dans la méthode selon l'invention, le sujet est prédit comme répondeur à un traitement avec au moins un agent bloquant HER2, en particulier le trastuzumab, si le nucléotide au niveau du site polymorphe rs3746083 est un nucléotide C pour les deux allèles (les deux copies) du gène codant la protéine tristétraproline chez ledit sujet.

Ladite méthode de prédiction selon l'invention peut en outre comprendre l'étape suivante :
- l'affectation dudit sujet à un groupe non-répondeur si le nucléotide au niveau du site polymorphe rs3746083 n'est pas un nucléotide C pour au moins un allèle (au moins une copie), en particulier les deux allèles (les deux copies) du gène codant la protéine tristétraproline, notamment si le nucléotide au niveau du site polymorphe rs3746083 est un nucléotide T pour au moins un allèle (au moins une copie), en particulier les deux allèles (les deux copies) du gène codant la protéine tristétraproline chez ledit sujet, ou
- l'affectation dudit sujet à un groupe répondeur si le nucléotide au niveau du site polymorphe rs3746083 est un nucléotide C pour les deux allèles (les deux copies) du gène codant la protéine tristétraproline chez ledit sujet.

La méthode selon l'invention, peut comprendre la détection d'au moins un polymorphisme (en particulier d'un polymorphisme d'un seul nucléotide, encore nommé SNP) en déséquilibre de liaison avec le polymorphisme rs3746083. De tels polymorphismes en déséquilibre de liaison avec le polymorphisme rs3746083 peuvent être identifiés par toute technique bien connue de l'Homme du Métier.

Par exemple, l'identification de polymorphisme en déséquilibre de liaison avec le polymorphisme rs3746083 peut comprendre: (a) l'amplification d'un fragment de la région génomique comprenant ou entourant le polymorphisme rs3746083 d'une pluralité de sujets; (b) l'identification de seconds polymorphismes dans la région génomique comprenant ou flanquant ledit polymorphisme rs3746083; (c) l'analyse du déséquilibre de liaison entre ledit polymorphisme rs3746083 et lesdits seconds polymorphismes, et (d) la sélection desdits seconds polymorphismes en déséquilibre de liaison avec ledit polymorphisme rs3746083.

On entend par « échantillon biologique » au sens de la présente invention, tout échantillon biologique issu d'un sujet. Ce terme inclut tout fluide biologique, échantillon de tissus, de cellules, d'organes, biopsies, toute culture de tissus ou cellules dérivés de ceux-ci.

En particulier, ledit échantillon biologique peut être un échantillon biologique « pathologique » (caractéristique d'une pathologie), tel qu'un échantillon de tissu(s) ou cellule(s) pathologique(s), en particulier cancéreux et tout particulièrement HER2-positif(s).

Le terme « échantillon biologique » inclut également les échantillons qui ont été manipulés, traités notamment par des réactifs, par solubilisation, par enrichissement de certains éléments. Ainsi, l'échantillon biologique peut être traité avant son utilisation dans la méthode selon l'invention, par exemple, afin d'isoler et/ou concentrer des acides nucléiques ou des protéines, selon toutes techniques bien connues de l'Homme du Métier. A titre d'exemple de telles techniques, on peut citer les techniques de lyse (par exemple, mécanique, physique, chimique, etc), de concentration cellulaire, de dilution des acides nucléiques. Les acides nucléiques peuvent également être traités avec des enzymes ou autres traitements chimiques ou physiques pour produire des fragments de ceux-ci.

En particulier, ledit échantillon biologique utilisable pour l'étape i) comprend des acides nucléiques, tout particulièrement de l'ADN génomique dudit sujet et peut être choisi dans le groupe consistant en : un échantillon sanguin, tel qu'un échantillon de sang total, de sérum, de plasma, un échantillon de salive, un échantillon de liquide séminal et un échantillon d'urine.

En particulier, ledit échantillon biologique utilisable pour l'étape ii) comprend des protéines dudit sujet et peut être choisi dans le groupe consistant en : un échantillon sanguin, un échantillon de tissu(s) ou cellule(s), en particulier de tissu(s) ou cellule(s) mammaire(s), tout particulièrement de tissu(s) ou cellule(s) cancéreux et encore plus particulièrement de tissu(s) ou cellule(s) cancéreux HER2-positif.

Le terme « sujet » tel qu'utilisé dans la présentedescription, se réfère à tout individu, patient, en particulier tout être humain pour lequel la prédiction, le pronostic, le diagnostic ou la thérapie est souhaité. En particulier, dans la présente invention, le sujet est une femme et tout particulièrement une femme atteinte d'un cancer du sein HER2-positif.

Ledit sujet peut être atteint d'une pathologie et/ou de troubles pour lesquels un traitement par au moins un agent bloquant HER2 est bénéfique, incluant les tumeurs bénignes et malignes. En particulier, ledit sujet est atteint d'une pathologie et/ou de troubles liés à HER2, tout particulièrement d'une pathologie HER2-positive et encore plus particulièrement d'un cancer HER2-positif.

On entend par « pathologie et/ou troubles liés à HER2 » au sens de la présente description, toute pathologie et/ou trouble pour lequel un traitement avec au moins un agent bloquant HER2 est bénéfique.

On entend par « pathologie HER2-positive, en particulier cancer HER2-positif » au sens de la présente description, toute pathologie, en particulier tout cancer, dans laquelle la protéine HER2 est surexprimée, à savoir, présente un niveau anormal d'expression dans une cellule, tissu ou organe du sujet atteint de cette pathologie par rapport au niveau d'expression dans une cellule, tissu ou organe d'un sujet sain ou non atteint de cette pathologie.

Les cancers HER2-positifs incluent notamment les cancers du sein, de l'ovaire, du côlon, du pancréas, de la prostate, de l'estomac, de l'endomètre et les cancers bronchiques non à petites cellules (CBNP). En particulier, dans la présente invention, le cancer HER2-positif est un cancer du sein HER2-positif.

Les sujets atteints d'une pathologie HER2-positive, en particulier d'un cancer HER2-positif, peuvent être identifiés en utilisant toute technique bien connue de l'Homme du Métier telles que des techniques électrophorétiques et immunologiques utilisant des anticorps dirigés contre HER2 ou encore des techniques d'hybridation chromogénique *in situ* (CISH®) permettant de détecter l'amplification du gène codant HER2, notamment sur des coupes de tissus fixés inclus en paraffine. A titre d'exemple, on peut citer le kit Zymed's Spot-Light® HER2 CISHTM (commercialisé par Zymed® laboratories) permettant de détecter l'amplification du gène HER2 dans des sections de tissu fixées dans du formol et incorporées dans de la paraffine (formalinfixed paraffin embedded tissue, FFPE) par l'hybridation chromogénique *in situ* (Chromogenic *In Situ* Hybridization, CISH™).

La méthode selon l'invention permet de distinguer les sujets répondeurs des sujets non-répondeurs à un traitement avec au moins un agent bloquant HER2, le trastuzumab.

On entend par « sujet répondeur à un traitement avec au moins un agent bloquant HER2, le trastuzumab» au sens de la présente invention, tout sujet qui montre un soulagement cliniquement significatif d'une pathologie et/ou de troubles liés à HER2, en particulier d'une pathologie HER2-positive, tout particulièrement d'un cancer HER2-positif et encore plus particulièrement au sens de la présente invention d'un cancer du sein HER2-positif, lorsqu'il est traité avec ledit agent, selon les critères RECIST (recommandations pour l'évaluation de la réponse tumorale dans les tumeurs solides, *« Response Evaluation Criteria in Solid Tumors »).*

On entend par « sujet non-répondeur à un traitement avec au moins un agent bloquant HER2, le trastuzumab», tout sujet qui ne montre pas de soulagement cliniquement significatif d'une pathologie et/ou de troubles liés à HER2, en particulier d'une pathologie HER2-positive, tout particulièrement d'un cancer HER2-positif et encore plus particulièrement au sens de la présente invention d'un cancer du sein HER2-positif, lorsqu'il est traité avec ledit agent, selon les critères RECIST (recommandations pour l'évaluation de la réponse tumorale dans les tumeurs solides, *« Response Evaluation Criteria in Solid Tumors »*)*.*

L'étape ii) de détermination du taux de protéine tristétraproline dans un échantillon biologique d'un sujet peut être mise en oeuvre par toute technique bien connue de l'Homme du Métier.

De telles techniques peuvent comprendre la mise en contact d'un échantillon biologique avec un agent de liaison capable d'interagir sélectivement avec la protéine tristétraproline susceptible d'être présente dans l'échantillon biologique.

L'agent de liaison peut être un anticorps, en particulier polyclonal ou monoclonal et tout particulièrement monoclonal dirigé contre la protéine tristétraproline.

Ainsi, le taux de protéine tristétraproline peut être déterminé grâce à des techniques électrophorétiques et immunologiques utilisant des anticorps dirigés contre la protéine tristétraproline. A titre d'exemples, on peut citer les Western-blots, les tests enzymatiques tels qu'ELISA, les tests de type biotine/avidine, les tests radioimmunologiques, l'immunoélectrophorèse, l'immunoprécipitation. Ces techniques incluent généralement des marqueurs permettant de détecter la formation d'un complexe entre la protéine tristétraproline et l'agent de liaison, en particulier entre l'antigène et le ou les anticorps qui ont réagi avec celui-ci ; lesdits marqueurs pouvant être des marqueurs fluorescents, chimioluminescents, radioactifs, enzymatiques, des colorants ou autres.

Ladite méthode de prédiction selon l'invention peut comprendre en outre l'étape de :
iii) comparaison du taux de protéine tristétraproline dans un échantillon biologique dudit sujet déterminé à l'étape ii) avec au moins une valeur référence.

Cette étape iii) contribue à déterminer si un sujet est répondeur ou non-répondeur à un traitement avec au moins agent bloquant HER2, le trastuzumab.

Ladite valeur référence peut être notamment, sans s'y limiter :
- une valeur seuil référence ;
- la valeur moyenne du taux de protéine tristétraproline déterminée dans un tissu sain, ledit tissu sain avoisinant le tissu tumoral à partir duquel le taux de protéine tristétraproline a été déterminé à l'étape ii) ;
- la valeur moyenne du taux de protéine tristétraproline, déterminée dans un échantillon biologique équivalent, pour un groupe de sujets répondeurs à un traitement avec au moins un agent bloquant HER2, en particulier le trastuzumab ; ou
- la valeur moyenne du taux de protéine tristétraproline, déterminée dans un échantillon biologique équivalent, pour un groupe de sujets non-répondeurs à un traitement avec au moins un agent bloquant HER2, le trastuzumab.

On entend par « échantillon biologique équivalent » au sens de la présente invention, tout échantillon biologique correspondant physiologiquement à celui de l'étape ii). A titre d'exemple, lorsque l'échantillon biologique de l'étape ii) est un échantillon de tissus cancéreux mammaires, l'échantillon biologique équivalent peut être un échantillon de tissus mammaires, de préférence de la même région que celui de l'étape ii).

Ainsi, par exemple lorsque le taux de protéine tristétraproline déterminé à l'étape ii) est significativement moins élevé que la valeur référence, ladite valeur référence correspondant à la valeur moyenne du taux de protéine tristétraproline déterminée dans un tissu sain, ledit tissu sain avoisinant le tissu tumoral à partir duquel le taux de protéine tristétraproline à été déterminé à l'étape ii), alors le sujet est prédit comme non-répondeur audit au moins un agent bloquant HER2, le trastuzumab.

Ainsi, par exemple lorsque le taux de protéine tristétraproline déterminé à l'étape ii) est égal ou supérieur à la valeur référence, ladite valeur référence correspondant à la valeur moyenne du taux de protéine tristétraproline déterminée dans un tissu sain, ledit tissus sain avoisinant le tissu tumoral à partir duquel le taux de protéine tristétraproline à été déterminé à l'étape ii), alors le sujet est prédit comme répondeur audit au moins un agent bloquant HER2, le trastuzumab.

Ainsi, par exemple lorsque le taux de protéine tristétraproline déterminé à l'étape ii) est significativement moins élevé que la valeur référence, ladite valeur référence correspondant à la valeur moyenne du taux de protéine tristétraproline, déterminée dans un échantillon biologique équivalent, pour un groupe de sujets répondeurs à un traitement avec au moins un agent bloquant HER2, le trastuzumab, alors le sujet est prédit comme non-répondeur audit au moins un agent bloquant HER2, le trastuzumab.

Ainsi, par exemple lorsque le taux de protéine tristétraproline déterminé à l'étape ii) est égal ou supérieur à la valeur référence, ladite valeur référence correspondant à la valeur moyenne du taux de protéine tristétraproline, déterminée dans un échantillon biologique équivalent, pour un groupe de sujets répondeurs à un traitement avec au moins un agent bloquant HER2, en particulier le trastuzumab, alors le sujet est prédit comme répondeur audit au moins un agent bloquant HER2, le trastuzumab.

Par exemple lorsque le taux de protéine tristétraproline déterminé à l'étape ii) est significativement plus élevé que la valeur référence, ladite valeur référence correspondant à la valeur moyenne du taux de protéine tristétraproline, déterminée dans un échantillon biologique équivalent, pour un groupe de sujets non-répondeurs à un traitement avec au moins un agent bloquant HER2, le trastuzumab, alors le sujet est prédit comme répondeur audit au moins un agent bloquant HER2, le trastuzumab.

Par exemple lorsque le taux de protéine tristétraproline déterminé à l'étape ii) est égal ou inférieur à la valeur référence, ladite valeur référence correspondant à la valeur moyenne du taux de protéine tristétraproline, déterminée dans un échantillon biologique équivalent, pour un groupe de sujets non-répondeurs à un traitement avec au moins un agent bloquant HER2, le trastuzumab, alors le sujet est prédit comme non-répondeur audit au moins un agent bloquant HER2, en particulier le trastuzumab.

Un taux de protéine tristétraproline significativement plus élevé que la valeur référence peut correspondre à un taux supérieur à au moins 15%, au moins 20%, au moins 25%, au moins 30%, au moins 35% par rapport à la valeur référence.

Un taux de protéine tristétraproline significativement moins élevé que la valeur référence peut correspondre à un taux inférieur à au moins 15%, au moins 20%, au moins 25%, au moins 30%, au moins 35% par rapport à la valeur référence.

La méthode selon l'invention peut également comprendre la détermination du taux d'une protéine contrôle dans un échantillon biologique dudit sujet. Ladite protéine contrôle, peut notamment être une protéine dont le taux est constant chez les sujets répondeurs et non-répondeurs à un traitement avec au moins un agent bloquant HER2, en particulier le trastuzumab.

La méthode de prédiction selon l'invention peut également comprendre une étape de détermination d'au moins un paramètre additionnel utile pour la prédiction, notamment à partir d'un échantillon biologique dudit sujet. On entend par « paramètre additionnel utile pour la prédiction », tout paramètre qui peut ne pas être utilisé seul pour la prédiction mais qui a été décrit comme par exemple montrant des valeurs significativement différentes entre un sujet répondeur et un sujet non-répondeur à un traitement avec au moins un agent bloquant HER2 et qui peut être utile pour confirmer la prédiction déterminée par la méthode selon l'invention. Un tel paramètre additionnel utile pour la prédiction peut être :
- le niveau d'expression d'au moins un gène choisi dans le groupe consistant en GPR22 (« G protein-coupled receptor 22 », en particulier Référence Genebank : NM_005295), PEX19 (*« Peroxisomal biogenesis factor 19 »*, en particulier Référence Genebank : NM_002857), GRHL2 (*« Grainyhead-like 2* », en particulier Référence Genebank : NM_024915) et DERL1 (*« Derlin 1* », en particulier Référence Genebank : NM_024295), le gène codant HER2 (en particulier Référence Genebank : NM_004448).

Ladite méthode de prédiction selon l'invention peut comprendre en outre l'étape de :
iv) prédiction de la réponse dudit sujet à un traitement avec au moins un agent bloquant HER2, le trastuzumab, par l'évaluation des résultats obtenus à l'étape i) et/ou l'étape ii).

Selon un autre aspect, la description concerne un agent bloquant HER2, pour son utilisation en tant que médicament pour le traitement d'une pathologie et/ou de troubles liés à HER2, en particulier d'une pathologie HER2-positive, tout particulièrement d'un cancer HER2-positif et encore plus particulièrement d'un cancer du sein HER2-positif chez un sujet, ledit sujet étant prédit comme répondeur à un traitement avec ledit agent bloquant HER2 par la méthode de prédiction selon l'invention.

Selon présente invention, l'agent bloquant HER2 positif est le trastuzumab et la pathologie HER2-positive est le cancer du sein HER2-positif.

La présente description concerne également l'utilisation d'un agent bloquant HER2 pour la fabrication d'un médicament pour le traitement d'une pathologie et/ou de troubles liés à HER2, en particulier d'une pathologie HER2-positive, tout particulièrement d'un cancer HER2-positif et encore plus particulièrement d'un cancer du sein HER2-positif chez un sujet, ledit sujet étant prédit comme répondeur à un traitement avec ledit agent bloquant HER2 par la méthode de prédiction selon l'invention.

La présente description concerne également une méthode de traitement d'un sujet atteint d'une pathologie et/ou de troubles liés à HER2, en particulier d'une pathologie HER2-positive, tout particulièrement d'un cancer HER2-positif et encore plus particulièrement d'un cancer du sein HER2-positif, ladite méthode comprenant les étapes suivantes:
- a) prédiction de la réponse du sujet à un traitement avec au moins un agent bloquant HER2 par la mise en oeuvre de la méthode de prédiction selon l'invention; et
- b) administration d'une quantité thérapeutique d'un agent bloquant HER2 audit sujet prédit comme répondeur à un traitement avec au moins un agent bloquant HER2 à l'étape a).

On entend par « quantité thérapeutique » au sens de la présente invention, une quantité active et non-toxique d'un agent bloquant HER2.

Ces quantités thérapeutiques peuvent être déterminées par l'Homme du Métier par des tests de routine, comprenant l'évaluation de l'effet de l'administration d'au moins un agent bloquant HER2 sur les pathologies et/ou des troubles liés à HER2 que l'on cherche à traiter par l'administration dudit agent bloquant HER2, en particulier sur un cancer HER2-positif et tout particulièrement sur un cancer du sein HER2-positif.

Par exemple, ces tests peuvent être mis en oeuvre par l'analyse à la fois de l'effet quantitatif et qualitatif de l'administration de différentes quantités dudit agent bloquant HER2 (en particulier le trastuzumab) sur un ensemble de marqueurs (biologiques et/ou cliniques) caractéristiques de ces pathologies et/ou de ces troubles liés à HER2, notamment à partir d'au moins un échantillon biologique d'au moins un sujet.

La présente invention concerne également un produit comprenant :
- au moins un agent bloquant HER2 et
- au moins un autre agent de traitement d'une pathologie et/ou de troubles liés à HER2, en particulier d'une pathologie HER2-positive, tout particulièrement d'un cancer HER2-positif et encore plus particulièrement d'un cancer du sein HER2-positif,
en tant que produit de combinaison pour une utilisation simultanée, séparée ou étalée dans le temps pour le traitement d'une pathologie et/ou de troubles liés à HER2, en particulier d'une pathologie HER2-positive, tout particulièrement d'un cancer HER2-positif et encore plus particulièrement d'un cancer du sein HER2-positif chez un sujet, ledit sujet étant prédit comme répondeur à un traitement avec ledit agent bloquant HER2 par la méthode de prédiction selon l'invention, ledit agent bloquant HER2 étant du trastuzumab et ladite pathologie HER2 positive étant un cancer du sein HER2 positif.

La présente description concerne également l'utilisation :
- d'au moins un agent bloquant HER2 et
- d'au moins un autre agent de traitement d'une pathologie et/ou de troubles liés à HER2, en particulier d'une pathologie HER2-positive, tout particulièrement d'un cancer HER2-positif et encore plus particulièrement d'un cancer du sein HER2-positif,
pour la préparation d'un produit de combinaison pour une utilisation simultanée, séparée ou étalée dans le temps pour le traitement d'une pathologie et/ou de troubles liés à HER2, en particulier d'une pathologie HER2-positive, tout particulièrement d'un cancer HER2-positif et encore plus particulièrement d'un cancer du sein HER2-positif chez un sujet, ledit sujet étant prédit comme répondeur à un traitement avec ledit agent bloquant HER2 par la méthode de prédiction selon l'invention.

On entend par « agent de traitement d'une pathologie et/ou de troubles liés à HER2 » au sens de la présente invention, tout composé permettant de traiter une pathologie et/ou un trouble lié à HER2.

A titre d'exemples, ledit autre agent de traitement d'une pathologie et/ou de troubles liés à HER2, en particulier d'une pathologie HER2-positive, tout particulièrement d'un cancer HER2-positif et encore plus particulièrement d'un cancer du sein HER2-positif, peut être :
- un agent de chimiothérapie, tel que notamment dans le cas du cancer du sein les taxanes (notamment le docetaxel ou Taxotere®), doxorubicine, 5-fluorouracil, epirubicin, cyclophosphamide,
- un agent de thérapie hormonale, tel que notamment dans le cas du cancer du sein le tamoxifène, ou
- un agent de radio-thérapie.

Ledit agent bloquant HER2 peut être présent dans les médicaments et produits de combinaison selon l'invention en une quantité thérapeutique.

Ledit agent bloquant HER2 et ledit autre agent de traitement d'une pathologie et/ou de troubles liés à HER2 peuvent être présents dans les produits de combinaison selon l'invention selon un rapport molaire allant de 100/1 à 1/100.

Les médicaments et produits de combinaison selon l'invention sont administrables par différentes voies, notamment en fonction du type d'agent bloquant HER2. A titre d'exemples de voies d'administration utilisables pour les médicaments et produits de combinaison selon l'invention, on peut citer la voie orale, rectale, cutanée, pulmonaire, nasale, sublinguale et la voie parentérale.

Les médicaments et produit de combinaison selon l'invention peuvent comprendre en outre un véhicule pharmaceutiquement acceptable.

On entend par « véhicule pharmaceutiquement acceptable » au sens de la présente invention, toute matière qui est appropriée à une utilisation dans un produit pharmaceutique.

A titre d'exemples de véhicule pharmaceutiquement acceptable, on peut citer le lactose, l'amidon éventuellement modifié, la cellulose, l'hydroxypropyl cellulose, l'hydroxypropylmethyl cellulose, le mannitol, le sorbitol, le xylitol, le dextrose, le sulfate de calcium, le phosphate de calcium, le lactate de calcium, les dextrates, l'inositol, le carbonate de calcium, la glycine, la bentonite, la polyvinylpyrrolidone et leurs mélanges.

Les médicaments et produits de combinaison selon l'invention peuvent comprendre une teneur en véhicule pharmaceutiquement acceptable allant de 5 à 99% en poids, notamment de 10 à 90% en poids et en particulier de 20 à 75% en poids par rapport au poids total des médicaments ou des produits de combinaison selon l'invention.

Selon un autre aspect, La description concerne un kit de prédiction de la réponse d'un sujet à un traitement avec au moins un agent bloquant HER2 comprenant :
- des moyens d'identification du nucléotide au niveau du site polymorphe rs3746083; et/ou
- des moyens de détermination du taux de protéine tristétraproline ; et éventuellement
- des instructions d'utilisation desdits moyens pour prédire la réponse d'un sujet à un traitement avec au moins un agent bloquant HER2.

Les moyens d'identification du nucléotide au niveau du site polymorphe rs3746083 peuvent être des amorces et/ou des sondes d'acides nucléiques permettant l'identification du nucléotide au niveau du site polymorphe rs3746083 par séquençage, amplification et/ou hybridation.

En particulier, les moyens d'identification du nucléotide au niveau du site polymorphe rs3746083 peuvent être choisis dans le groupe consistant en :
- des amorces spécifiques et des réactifs permettant de séquencer de l'ADN génomique, de l'ADNc ou de l'ARN d'un sujet comprenant le nucléotide au niveau du site polymorphe rs3746083 ;
- des amorces spécifiques et des réactifs permettant d'amplifier de l'ADN génomique, de l'ADNc ou de l'ARN d'un sujet comprenant le nucléotide au niveau du site polymorphe rs3746083. En particulier, de telles amorces d'acides nucléiques sont capables de s'hybrider spécifiquement avec des parties de l'ADN génomique, de l'ADNc ou de l'ARN d'un sujet qui flanquent le nucléotide au niveau du site polymorphe rs3746083 ;
- des sondes d'acides nucléiques spécifiques de l'allèle C, de l'allèle T, de l'allèle A, de l'allèle G du polymorphisme localisé en position rs3746083 sur le génome humain et des réactifs permettant de détecter la formation d'hybrides spécifiques entre les séquences d'acides nucléiques complémentaires qui servent à détecter le polymorphisme.

En particulier, les moyens de détermination du taux de protéine tristétraproline peuvent être des anticorps dirigés contre la protéine tristétraproline, en particulier utilisables en ELISA (« enzyme-linked immunosorbent assay ») ou RIA (« radioimmunoassay »).

Le kit de prédiction selon l'invention peut comprendre en outre des éléments supplémentaires tels que des tampons, des réactifs, des marqueurs, des échantillons de contrôle.

Les instructions d'utilisation desdits moyens pour prédire la réponse d'un sujet à un traitement avec au moins un agent bloquant HER2 permettent notamment d'interpréter les résultats obtenus après identification du nucléotide au niveau du site polymorphe rs3746083 et/ou détermination du taux de protéine tristétraproline dans un échantillon biologique d'un sujet.

A titre d'exemple, lesdites instructions peuvent indiquer que l'absence de nucléotide C au niveau du site polymorphe rs3746083, pour au moins un allèle (au moins une copie), en particulier les deux allèles (les deux copies) du gène codant la protéine tristétraproline chez un sujet est indicative d'un risque de non-réponse dudit sujet à un traitement avec au moins un agent bloquant HER2, en particulier le trastuzumab. Ledit sujet est alors prédit comme non-répondeur audit traitement.

Selon un autre aspect, la présente invention a pour objet une méthode *in vitro* ou *ex vivo* de pronostic ou de diagnostic d'un cancer, en particulier d'un cancer de mauvais pronostic, chez un sujet comprenant :
α) l'identification du nucléotide au niveau du site polymorphe rs3746083 367C>T sur la SEQ ID NO : 3, pour au moins un allèle (au moins une copie), en particulier les deux allèles (les deux copies) du gène codant la protéine tristétraproline dans un échantillon biologique dudit sujet.

Ledit cancer peut être tout cancer dans lequel la protéine tristétraproline est sous exprimée, à savoir, présente un niveau anormal d'expression dans une cellule, tissu ou organe du sujet atteint de ce cancer par rapport au niveau d'expression dans une cellule, tissu ou organe d'un sujet sain ou non atteint de ce cancer, tel qu'illustré dans les articles de Brennan *et al.* (2009).

En particulier, ledit cancer peut être compris dans le groupe consistant en les cancers du poumon, du sein, de l'utérus, de l'ovaire de la vulve, de la prostate, des testicules, de la trachée, de la thyroïde, du foie, de l'estomac, de l'intestin, du colon, du rectum, du pancréas, du rein, de la vessie et de la peau, tout particulièrement les cancers de la thyroïde, du poumon, de l'ovaire, de l'utérus, du sein, les adénomes et les adénocarcinomes et encore plus particulièrement le cancer du sein.

Dans la méthode de pronostic ou de diagnostic de l'invention, ledit cancer est un cancer du sein de mauvais pronostic.

Dans la méthode de pronostic ou de diagnostic selon l'invention, l'absence de nucléotide C au niveau du site polymorphe rs3746083, pour au moins un allèle (au moins une copie), en particulier les deux allèles (les deux copies) du gène codant la protéine tristétraproline est indicative du risque que le sujet soit atteint d'un cancer, en particulier d'un cancer de mauvais pronostic et tout particulièrement d'un cancer du sein de mauvais pronostic.

Dans la méthode de pronostic ou de diagnostic selon l'invention, la présence d'un nucléotide T au niveau du site polymorphe rs3746083, pour au moins un allèle (au moins une copie), en particulier les deux allèles (les deux copies) du gène codant la protéine tristétraproline est indicative du risque que le sujet soit atteint d'un cancer, en particulier d'un cancer de mauvais pronostic et tout particulièrement d'un cancer du sein de mauvais pronostic.

Ainsi, dans la méthode selon l'invention, l'identification d'une hétérozygotie (T/C) au niveau du site polymorphe rs3746083 chez un sujet (présence d'un nucléotide T au niveau du site polymorphe rs3746083, pour un allèle du gène codant la protéine tristétraproline et présence d'un nucléotide C au niveau du site polymorphe rs3746083, pour l'autre allèle du gène codant la protéine tristétraproline) est indicative du risque que le sujet soit atteint d'un cancer, en particulier d'un cancer de mauvais pronostic et tout particulièrement d'un cancer du sein de mauvais pronostic.

La méthode de pronostic ou de diagnostic selon l'invention présente notamment les avantages suivants :
- Elle est simple et rapide. L'étape α) présente l'avantage de ne pas requérir le prélèvement d'un échantillon de la tumeur chez le sujet et d'éviter ainsi les différents risques et problèmes liés à la réalisation d'une biopsie. En effet, elle peut être mise en oeuvre à partir d'un simple échantillon sanguin d'un sujet de 5 à 10 ml par des techniques de biologie moléculaire simples. Elle nécessite une plateforme standard de biologie moléculaire déjà présente dans de nombreux hôpitaux. En outre, l'étape α) présente l'avantage de permettre l'obtention d'un résultat particulièrement simple à analyser ;
- Elle est fiable, reproductible et peu coûteuse.

La méthode de pronostic ou diagnostic selon l'invention peut également comprendre une étape de détermination d'au moins un paramètre additionnel utile pour le pronostic ou diagnostic, notamment à partir d'un échantillon biologique dudit sujet. On entend par « paramètre additionnel utile pour le pronostic ou diagnostic », tout paramètre qui ne peut pas être utilisé seul pour le pronostic ou diagnostic mais qui a été décrit comme par exemple montrant des valeurs significativement différentes entre un sujet atteint et un sujet non-atteint d'un cancer et qui peut être utile pour confirmer le pronostic ou diagnostic déterminé par la méthode selon l'invention. Un tel paramètre additionnel utile pour le pronostic ou diagnostic peut être :
- le niveau d'expression d'au moins un gène dont l'expression est modulée chez un sujet atteint dudit cancer, tel que le gène codant HER2 dans le cas des cancers HER2-positifs.

La description concerne également une méthode de traitement d'un sujet atteint d'un cancer, en particulier de mauvais pronostic, comprenant les étapes suivantes :
1) pronostic ou diagnostic d'un cancer chez ledit sujet par la mise en oeuvre de la méthode de pronostic ou de diagnostic selon l'invention ;
2) administration d'une quantité thérapeutique d'un agent anti-cancéreux audit sujet diagnostiqué comme atteint d'un cancer, en particulier de mauvais pronostic, à l'étape 1).

On entend par « agent anti-cancéreux », tout composé permettant de traiter un cancer et/ou un trouble lié à un cancer. A titre d'exemples, ledit agent anti-cancéreux peut être :
- un agent de chimiothérapie, tel que notamment dans le cas du cancer du sein les taxanes (notamment le docetaxel ou Taxotere®), doxorubicine, 5-fluorouracil, epirubicin, cyclophosphamide,
- un agent de thérapie hormonale, tel que notamment dans le cas du cancer du sein le tamoxifène, ou
- un agent de radio-thérapie.

En particulier, ledit agent anti-cancéreux est adapté au cancer diagnostiqué chez ledit sujet. Par exemple, lorsque le sujet est atteint d'un cancer du sein HER2-positif, ledit agent peut être le tamoxifène, le docetaxel....

La description concerne également un kit de pronostic ou de diagnostic d'un cancer, en particulier d'un cancer de mauvais pronostic, chez un sujet comprenant :
- des moyens d'identification du nucléotide au niveau du site polymorphe rs3746083; et éventuellement
- des instructions d'utilisation desdits moyens pour établir un pronostic ou un diagnostic d'un cancer, en particulier d'un cancer de mauvais pronostic, chez un sujet.

Les moyens d'identification du nucléotide au niveau du site polymorphe rs3746083 peuvent être des amorces et/ou des sondes d'acides nucléiques permettant l'identification du nucléotide au niveau du site polymorphe rs3746083 par séquençage, amplification et/ou hybridation.

En particulier, les moyens d'identification du nucléotide au niveau du site polymorphe rs3746083 peuvent être choisis dans le groupe consistant en :
- des amorces spécifiques et des réactifs permettant de séquencer de l'ADN génomique, de l'ADNc ou de l'ARN d'un sujet comprenant le nucléotide au niveau du site polymorphe rs3746083 ;
- des amorces spécifiques et des réactifs permettant d'amplifier de l'ADN génomique, de l'ADNc ou de l'ARN d'un sujet comprenant le nucléotide au niveau du site polymorphe rs3746083. En particulier, de telles amorces d'acides nucléiques sont capables de s'hybrider spécifiquement avec des parties de l'ADN génomique, de l'ADNc ou de l'ARN d'un sujet qui flanquent le nucléotide au niveau du site polymorphe rs3746083 ;
- des sondes d'acides nucléiques spécifiques de l'allèle C, de l'allèle T, de l'allèle A, de l'allèle G du polymorphisme localisé en position rs3746083 sur le génome humain et des réactifs permettant de détecter la formation d'hybrides spécifiques entre les séquences d'acides nucléiques complémentaires qui servent à détecter le polymorphisme.

Lesdites instructions d'utilisation desdits moyens pour établir un pronostic ou un diagnostic d'un cancer, en particulier d'un cancer de mauvais pronostic, chez un sujet, permettent notamment d'interpréter les résultats obtenus après identification du nucléotide au niveau du site polymorphe rs3746083.

A titre d'exemple, lesdites instructions peuvent indiquer que l'absence de nucléotide C au niveau du site polymorphe rs3746083, pour au moins un allèle (au moins une copie), en particulier les deux allèles (les deux copies) du gène codant la protéine tristétraproline est indicative du risque que le sujet soit atteint d'un cancer, en particulier d'un cancer de mauvais pronostic.

D'autres avantages et caractéristiques de l'invention apparaitront au regard des exemples qui suivent.

Ces exemples sont donnés à titre illustratif et non limitatif.

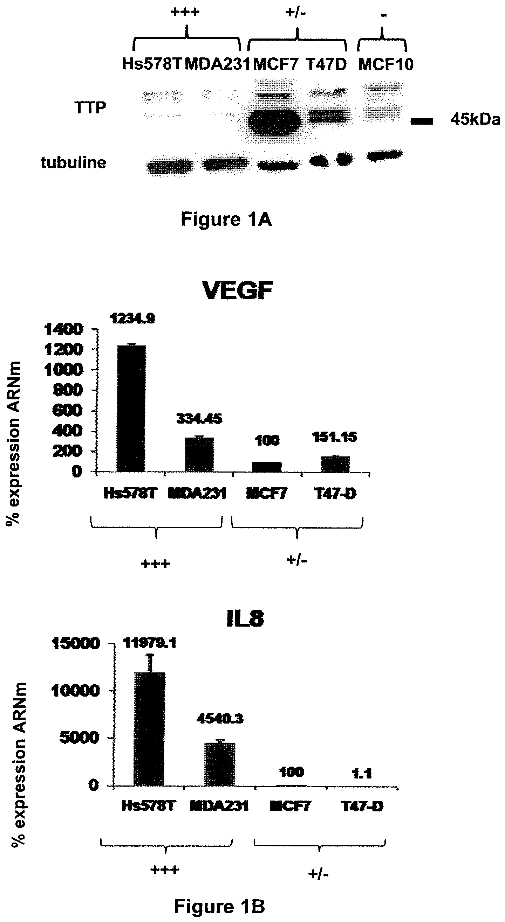

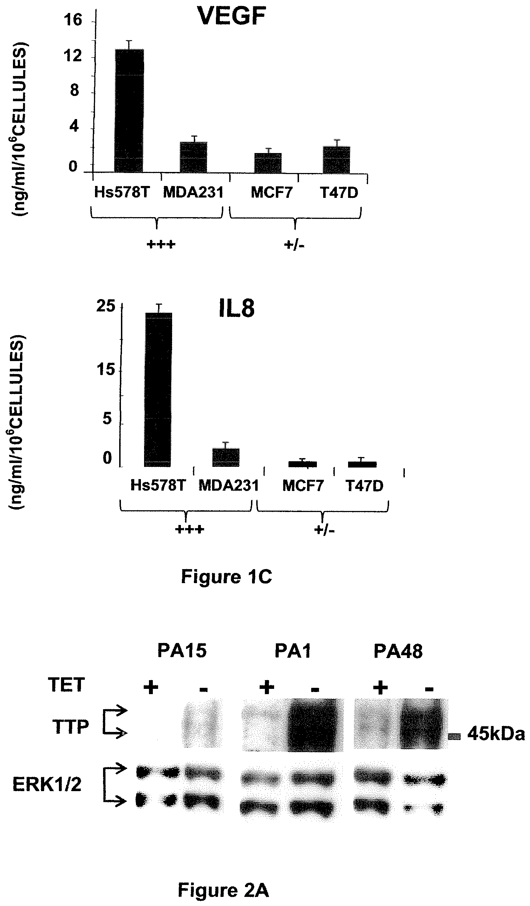

La Figure 1 (A-C) représente l'expression de TTP (tristétraproline), VEGF (facteur de croissance de l'endothélium vasculaire ou « Vascular Endothelial Growth Factor ») et IL8 (interleukine 8) dans des lignées cellulaires du cancer du sein. (A). Immunoblot des protéines totales TTP dans des lysats de cancer du sein et dans une lignée de cellules mammaires immortalisées. (+++) Lignées cellulaires de cancer du sein très agressives; (+/-) lignées cellulaires de cancer du sein non agressives; (-) lignée de cellules de sein non tumorales. (B) et (C) niveau d'ARNm et de protéines sécrétées VEGF et IL8 déterminé respectivement par qPCR et ELISA.

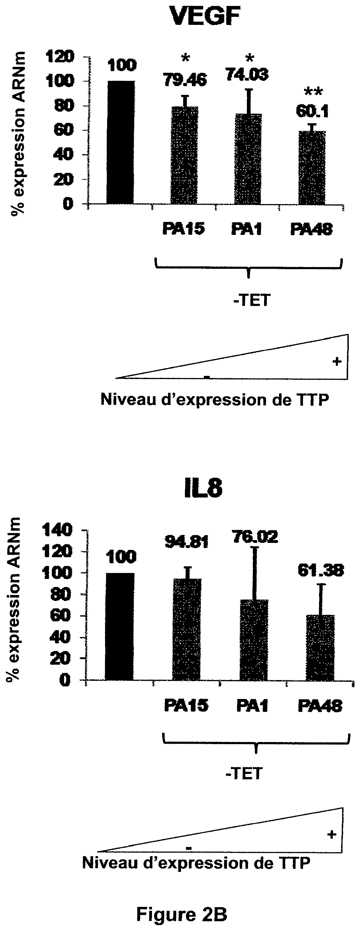

La Figure 2 (A-B) représente des clones inductibles TTP-MDA231. (A) Différents niveaux d'induction de protéines TTP dans trois clones (niveaux faibles de TTP = PA15, niveaux élevés de TTP= PA1 et PA48) après le retrait de tétracycline. (B) PCR quantitative des quantités totales d'ARNm du VEGF et d'IL8 dans les clones TTP-MDA231; *= p<0.05, **=p<0.01.

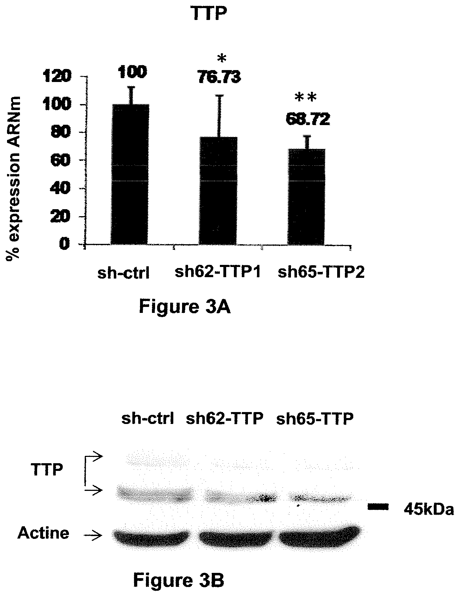

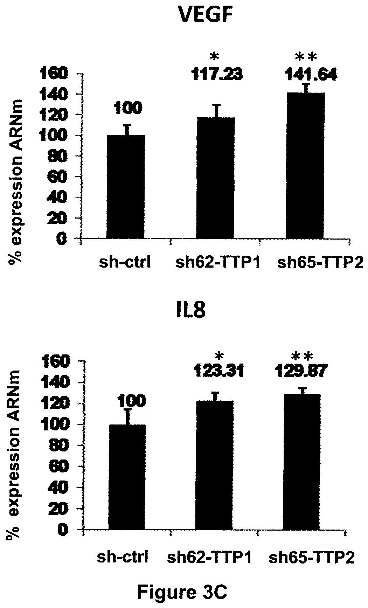

La Figure 3 (A-C) représente les clones stables MCF7 obtenus par transfection des séquences shCTRL, sh62-TTP1 et sh65-TTP2. (A) PCR quantitative des taux d'ARNm totaux TTP dans trois clones différents. (B) taux de protéine TTP correspondante détectée par immunoblot. (C) PCR quantitative des niveaux d'ARNm VEGF et IL8 dans les trois clones.*= p<0.05, **=p<0.01.

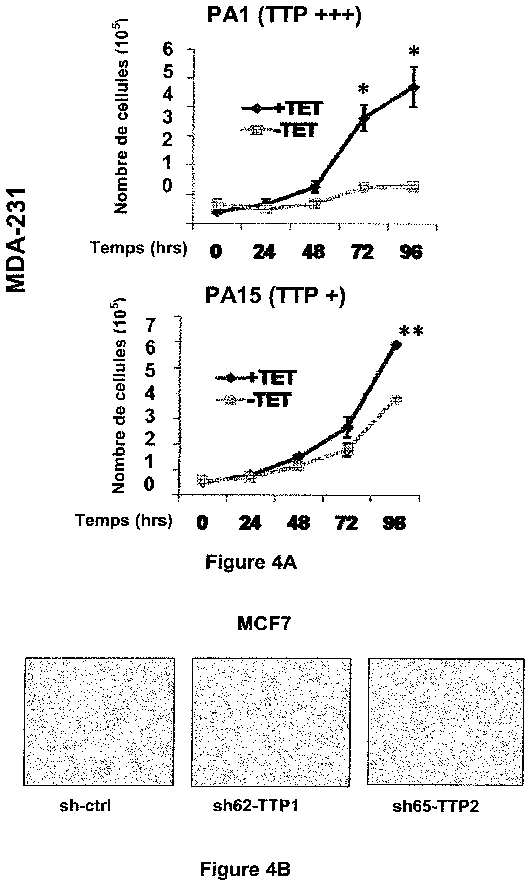

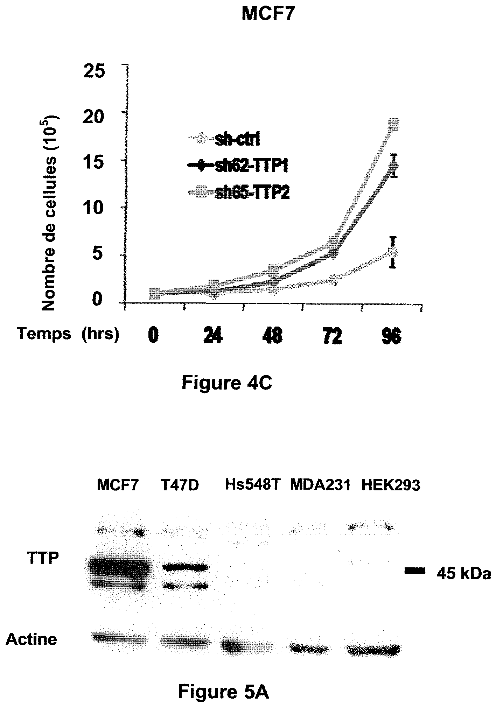

La Figure 4 (A-C) représente l'effet de l'expression de TTP sur la prolifération des clones inductibles MDA231-TTP et des clones stables shTTP-MCF7. (A) test de prolifération des clones inductibles TTP-MDA231 PA1 et PA15 en présence ou en absence de tétracycline. Les niveaux d'expression de TTP après l'induction sont indiqués (TTP + + + = niveaux élevés et TTP + = faible niveau. (B) morphologie cellulaire. (C) test de prolifération de deux clones stables shTTP-MCF7 par rapport à shCTRL.

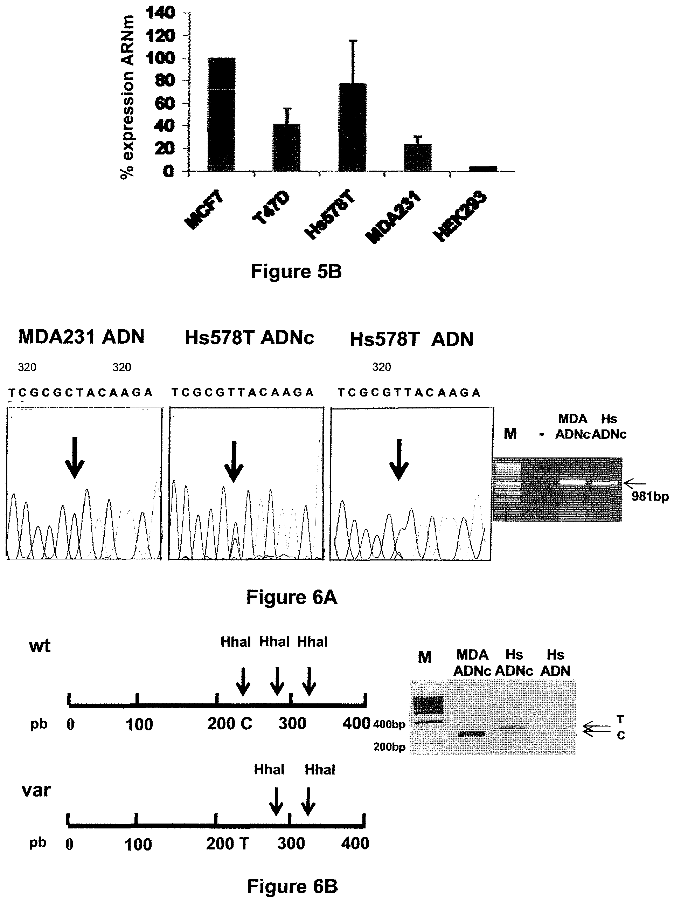

La Figure 5 (A-B) représente l'expression comparative de TTP dans des lignées cellulaires du cancer du sein. (A) Extraits protéiques de cancer du sein et lignées cellulaires HEK293 analysés par immunoblotting. (B) Niveaux d'ARNm correspondant dans les mêmes lignées cellulaires, quantifiés par PCR quantitative.

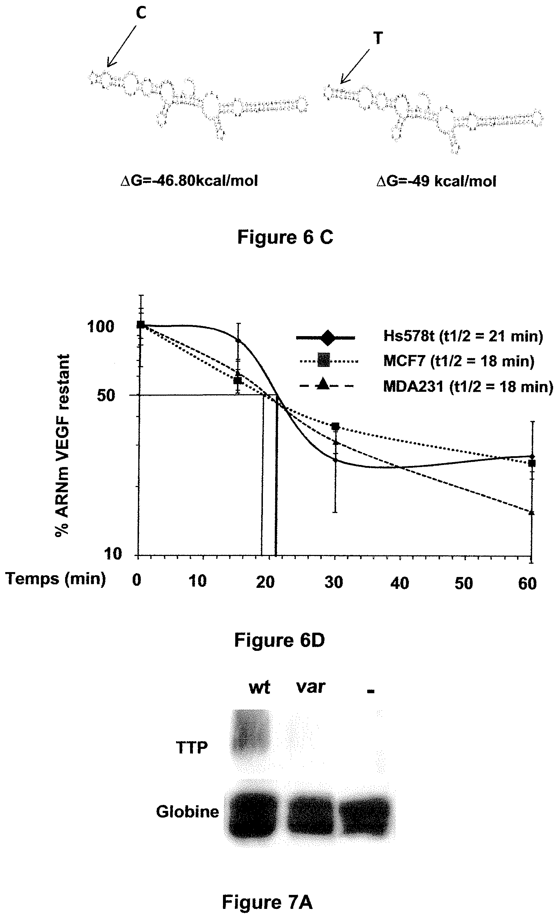

La Figure 6 (A-D) représente l'identification du nucléotide au niveau du site polymorphe rs3746083 (en particulier, la détection d'un nucléotide T au niveau du site polymorphe rs3746083) dans les cellules Hs578T. (A) chromatogrammes des séquences et analyse PCR de la région codant TTP de l'ADN génomique et de l'ADNc obtenus à partir de cellules MDA231 et Hs578T. (B) Schéma et analyse de digestion des fragments PCR clivés avec l'enzyme HhaI. (C) structure secondaire d'ARNm prédite par le logiciel MFOLD pour le type sauvage (« wild-type ») (présence d'un nucléotide C au niveau du site polymorphe rs3746083) et la séquence variante (présence d'un nucléotide T au niveau du site polymorphe rs3746083). (D) Temps de demi-vie de l'ARNm TTP déterminé par cinétiques au DRB (5.6-dichloro-1-D-ribofuranosylbenzimidazole) du des cellules MCF7, Hs578t et MDA231.

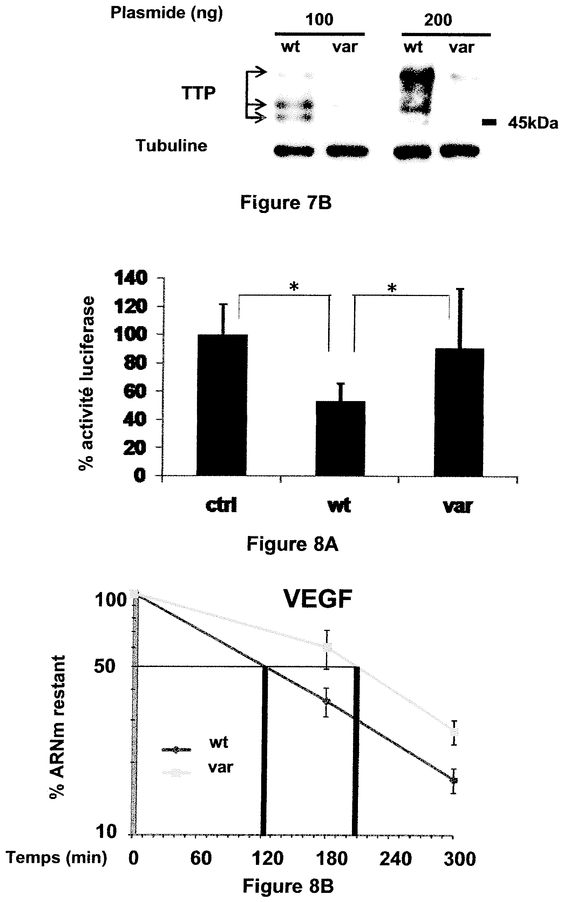

La Figure 7 (A-B) représente l'effet de TTP de type sauvage (wt) (présence d'un nucléotide C au niveau du site polymorphe rs3746083) ou du variant TTP (var) (présence d'un nucléotide T au niveau du site polymorphe rs3746083) sur la traduction. (A) Traduction *in vitro* des plasmides d'expression TTP-Myc de type sauvage (wt) et du variant (var). (B) Quantités croissantes des plasmides Trex (50 ng, 100 ng, 200 ng) portant les séquences TTP de type sauvage et du variant qui ont été transfectées dans les cellules HEK293. Les lysats protéiques ont été analysés par immunoblot. Deux préparations d'ADN indépendantes ont été utilisées à des concentrations de 200 ng. La comparaison entre les échantillons a été réalisée après le calcul de l'efficacité de la transfection (comme expliqué dans la section Matériels et Méthodes)

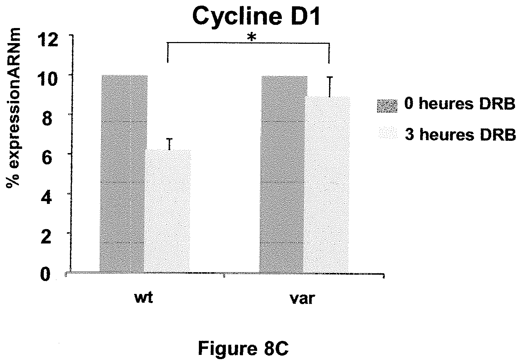

La Figure 8 (A-C) représente l'effet fonctionnel de l'allèle variant TTP (allèle T, présence d'un nucléotide T au niveau du site polymorphe rs3746083) sur la stabilité de gènes cibles (A) dosage de l'activité du gène rapporteur luciférase couplé à la région 3'UTR de l'ARNm du VEGF après transfection d'un vecteur vide, d'un vecteur d'expression contenant le gène TTP de type sauvage ou le gène TTP variant (présence d'un nucléotide T au niveau du site polymorphe rs3746083). Les moyennes d'activité relative luciférase ont été calculées sur la base de quatre transfections indépendantes. (B) cinétique DRB et mesure du temps de demi-vie de l'ARNm VEGF endogène après transfection avec les constructions TTP de type sauvage ou du variant. (C) Effet de TTP de type sauvage et du variant TTP sur l'ARNm endogène de la Cycline D1, après trois heures de traitement DRB. La quantité d'ARNm au temps 0 est considérée comme la valeur de référence (100 %).

### EXEMPLES

### I. MATERIELS ET METHODES

### I.1 Constructions de plasmides

Pour l'approche TET-Off (TET : tetracycline) dans les cellules MDA231, la construction pREV-TTP a été obtenue en insérant un fragment d'ADN de 1 kb correspondant à la région codante de l'ADNc de TTP (Essafi-Benkhadir *et al.,* 2007) dans les sites de restriction HindIII du plasmide pREV (Clontech). Les constructions pcDNA4/TO/myc-HysA (Trex-TTP) contenant la séquence de type sauvage (présence d'un nucléotide C au niveau du site polymorphe rs3746083) et la séquence variante de TTP humaine (présence d'un nucléotide T au niveau du site polymorphe rs3746083) ont été générées en amplifiant une région de 1kb directement à partir d'ADNc de cellules Hs578T avec des amorces sens (5'CCACTCTCGGCCGACACCCC-3') et anti-sens (5'- GTCACTCAGAAACAGAGATGCG-3') et en insérant le fragment dans le vecteur pCR2.1-TOPO (Invitrogen). Les fragments d'ADNc TTP ont été ensuite insérés dans le vecteur pcDNA4/TO/myc-HysA (Invitrogen) au niveau du site de restriction EcoRI. Deux préparations de plasmides indépendantes ont été obtenues pour chaque construction.

### I.2 Préparation de l'ARN et analyse par PCR quantitative

L'ARN total a été extrait au réactif TRIzol (Invitrogen). Deux microgrammes d'ARN total ont été utilisés pour la transcription inverse, en utilisant le kit Superscript First-Strand Synthesis System (QIAGEN, Hilden, Germany), avec des amorces Oligo(dT) pour amorcer la synthèse du premier brin d'ADN. Pour la PCR en temps réel, les kits Taqman Gene expression assays (Applied biosystems) et qPCR core (Eurogentec) ont été utilisés. Pour calculer l'expression relative des ARNm de TTP, VEGF et d'IL8 dans les lignées cellulaires, la méthode 2[ddC(T)] a été utilisée (Schmittgen, 2008) et la normalisation a été réalisée avec le gène RPLP0 (Essafi-Benkhadir *et al.,* 2007). Pour calculer la stabilité de l'ARNm, 25 µg/mL de 5,6-dichloro-1--D-ribofuranosylbenzimidazole (DRB) ont été ajoutés aux cellules de cancer du sein en culture et l'ARN en a été extrait à différents temps. La quantité relative de chaque ARNm au temps 0 avant l'addition de DRB dans le milieu de culture a été fixée à 100%.

### I.3 Culture cellulaire, transection et test luciférase

Les lignées de cellules de cancer du sein MDA231, Hs578T, MCF7, T47D, MCF10 et la lignée de cellule de rein embryonnaire humain HEK293 ont été mises en culture comme décrit précédemment (Essafi-Benkhadir *et al.,* 2007, Eckert *et al.,* 2004). Les cellules RAW264.7 ont été cultivées dans du milieu Eagle modifié de Dulbecco, supplémenté de sérum de veau foetal dans une atmosphère humidifiée à 5 % de CO₂ et à 37 °C. Elles ont été stimulées avec du lipopolysaccharide (LPS) (Sigma- Aldrich) à une concentration de 10 ng/mL. La validation de l'anticorps TTP a été réalisée par l'analyse immunoblot sur des extraits protéiques de cellules RAW264.7 stimulées avec du LPS et de cellules HEK293 transfectées avec la forme TTP humaine (Lai *et al.,* 1999). Les clones stables ont été obtenus en transfectant le plasmide pREV-TTP avec de la Lipofectamine™ 2000 (Invitrogen) et les clones résistants à l'hygromycine ont été criblés par immunoblot après la suppression de la tétracycline du milieu de culture. L'inactivation de TTP dans les cellules MCF7 a été obtenue en transfectant les cellules avec de la lipofectamine et des plasmides lentivirus MISSION™ shRNA (SIGMA). La sélection des clones résistants a été réalisée en ajoutant de la puromycine au milieu de culture et le criblage a été effectué par qPCR sur les clones sélectionnés.

Le test fonctionnel du polymorphisme rs3746083 de TTP sur la traduction a été réalisé sur les cellules HEK293 transfectées avec les plasmides correspondant aux types sauvages (wt) (présence d'un nucléotide C au niveau du site polymorphe rs3746083) et variant de TTP (var) (présence d'un nucléotide T au niveau du site polymorphe rs3746083) en utilisant la méthode de transfection au phosphate de calcium (Essafi-Benkhadir *et al.,* 2007). Le test a été effectué en double avec différentes quantités de pcDNA4/TO/myc-HysA portant la séquence sauvage et la séquence variante de TTP (deux préparations indépendantes pour chaque construction). En même temps, 300 ng de plasmide exprimant le gène rapporteur luciférase (plasmide pGL3) ont été co-transfectés en tant que contrôles indépendants de l'efficacité de la transfection dans chaque puits. Le test a été réalisé comme décrit précédemment (Essafi-Benkhadir *et al.,* 2007). L'efficacité de la transfection a été calculée à partir du taux de luciférase normalisé à la quantité de protéines. Seules les cellules qui ont montré le même niveau d'efficacité de transfection (différence < 20 %) ont été analysées. La solution de lyse de Laemmli a été ajoutée aux cellules. Les protéines des extraits ont été séparées par SDS-PAGE puis transférées sur une membrane de difluorure de polyvinylidène (Immobilon-P; Millipore, Billerica, MA). Les protéines immuno-réactives sont révélées avec le système de détection par chimioluminescence amélioré (ECL; Pierce Chemical, Rockford, IL).

### I.4 Traitement à la phosphatase alcaline d'intestin de veau (Calf Intestine Alkaline Phosphatase ou CIAP)

Pour les expériences avec la CIAP (New England Biolabs, Ipswich, MA), les cellules MDA231 ont été privées de tétracycline pendant 24 heures avant l'analyse. Les cellules ont été ensuite lysées dans un tampon de lyse (1% de Triton X-100, 50 mM de Tris, pH 8.5, 100 mM de NaCl et 0.5 mM d'EDTA). La CIAP (35 U) a été ajoutée aux lysats pendant 1 heure à 37 °C. La réaction a été arrêtée en y ajoutant le tampon de lyse de Laemmli.

### I.5 Mesure de la sécrétion de VEGF et IL-8

La présence de VEGF et d'IL-8 dans les surnageants cellulaires a été mesurée en utilisant le kit de dosage d'immunoadsorption par enzyme liée (en anglais kit ELISA, pour enzyme-linked immunosorbent assay) pour le VEGF et l'IL-8 humains (Pierce Biotechnology, Rockford, IL).

### I.6 Patients et études d'association

92 femmes avec un cancer du sein et 89 femmes contrôles ont été analysées pour la présence d'un nucléotide T au niveau du site polymorphe rs3746083. Toutes les patientes atteintes de cancer avaient des tumeurs très agressives présentant une amplification du gène HER2 (HER2-positif). Elles ont toutes été traitées avec l'Herceptine® (trastuzumab), un anticorps monoclonal dirigé contre HER2. L'ADN génomique a été extrait des leucocytes sanguins périphériques en utilisant des techniques standards. Pour le SNP NM_003407.2: 367C>T (présence d'un nucléotide T au niveau du site polymorphe rs3746083), un simple amplicon de 400 pb a été généré en utilisant les amorces 103F (5'-CGACCATGGAGGGACTGAG-3') et 103R (5'-GCCCTGGAGGTAGAACTTGT), et en suivant les conditions de PCR suivantes : 200 ng d'ADN génomique, 50 pM de chaque amorce, 200 µM de chaque dNTP, du tampon 1X, 0,9 unité de Taq Polymérase (AmpliTaq Gold - Applied Biosystem, Foster City, CA, USA) et 1,5 mM de MgCl2, dans un volume de réaction de PCR de 50 µL. La dénaturation initiale à 95 °C pendant 10 minutes est suivie de 35 cycles d'amplification à 95°C pendant 45 secondes, 62 °C pendant 45 secondes, 72 °C pendant 45 secondes, puis d'une extension finale à 72 °C pendant 10 minutes. La présence ou l'absence de l'allèle T du variant au locus rs3746083 (présence d'un nucléotide T au niveau du site polymorphe rs3746083 ; NM_003407.2: 367C>T) est évaluée par digestion avec l'enzyme de restriction HhaI (en suivant les instructions du fabricant - New England BioLabs). Après la digestion enzymatique, les échantillons sont déposés sur un gel d'agarose à 3%.

### I.7 Analyse statistique

Les fréquences des allèles sont estimées à partir des données de génotype. Le groupe de patientes et le groupe de contrôles sont comparés avec le test exact de Fisher en définissant *p*=0,05 comme le critère de pertinence statistique. L'équilibre de Hardy-Weinberg est testé avec le test du chi carré à la fois dans les groupes contrôles et les groupe de patientes. Pour l'étude clinique, la comparaison entre les paramètres de tumeurs ont été réalisées par ANOVA. Les estimations de survie ont été calculées en utilisant la méthode de Kaplan-Meier. Les différences entre les durées de survie ont été évaluées en utilisant le test du log-rank. Le test du chi carré a été utilisé pour déterminer les associations entre les génotypes des patients, la toxicité (hématologique, digestive) et la réponse à la thérapie basée sur le trastuzumab.

### II. RESULTATS

### II.1 Expression de TTP, VEGF et IL8 dans les lignées cellulaires de cancer du sein

Les taux de protéine TTP ont été analysés par immunoblot dans plusieurs lignées cellulaires de cancer du sein : MDA231, Hs578T, MCF7, T47D. MDA231 et Hs578T sont des lignées cellulaires très agressives, caractérisées par un phénotype mésenchymateux, un manque d'expression des récepteurs aux oestrogènes et à la progestérone et du récepteur HER2 (également appelé « phénotype triple négatif ») alors que MCF7 et T47D présentent un phénotype épithélial moins agressif et l'expression des récepteurs aux oestrogènes et à la progestérone sans amplification de la protéine HER2 (Eckert *et al.,* 2004). La taille prévue de la protéine TTP est de 35 kDa mais elle migre fréquemment sous la forme d'une bande de 45 à 47 kDa lors d'une analyse immunoblot SDS-PAGE conventionnelle utilisant des anticorps tant commerciaux (Suswam *et al.,* 2008, Al-Souhibani *et al.,* 2010) que « maison » (Essafi-Benkhadir *et al.* 2007). Il a été observé que deux lignées de cellules cancéreuses, MDA231 et Hs578T, n'exprimaient pas la protéine à la taille attendue, tandis que les cellules MCF7 et T47D l'exprimaient à des taux comparables à ceux de MCF10, une ligne de cellules mammaires non tumorigènes et immortalisées (Fig. 1A). Il a été examiné si le manque d'expression de protéine TTP pouvait se corréler avec une production plus élevée de facteurs angiogéniques tels que le VEGF et l'IL8 dans des cellules triples négatives (MDA231 et Hs578T) que dans des lignées cellulaires moins agressives (MCF7 et T47D). A cette fin, les taux d'ARNm de ces facteurs ont été déterminés par PCR quantitative en temps réel et les taux de protéines sécrétées par ELISA dans les quatre lignées. Les cellules MDA231 et Hs578T n'expriment pas la protéine TTP mais produisent une quantité élevée de VEGF et d'IL8 tant au niveau de l'ARNm que des protéines (Fig. 1B et 1C). Ces données sous-tendent une corrélation entre les taux de TTP et l'agressivité de cellules de tumeurs mammaires.

### II.2 La modulation de l'expression de TTP affecte l'expression de facteurs angiogéniques dans les lignées cellulaires mammaires cancéreuses peu ou très agressives

Pour étudier la corrélation entre la protéine TTP et les facteurs angiogéniques dans le cancer du sein, des clones de cellules MDA231/TET-OFF transfectées stablement avec le gène TTP étiqueté avec l'épitope Myc et inductible par la tétracycline ont été générées. Comme observé précédemment pour les cellules HeLa/TTP, le modèle TET-OFF convient mieux pour l'étude des gènes impliqués dans la prolifération cellulaire et évite la sélection de clones faux positifs (Suswam *et al.,* 2008). En présence de tétracycline l'expression de la protéine TTP est nulle. La suppression de la tétracycline du milieu permet l'induction de TTP. Un clone (PA15) à expression faiblement inductible et deux clones (PA1 et PA48) à expression hautement inductible de la protéine TTP ont été obtenus (Fig. 2A). Pour démontrer l'effet de la protéine TTP sur la production d'ARNm du VEGF et de l'IL8, une RT-PCR quantitative en temps réel a été réalisée et un effet dose-dépendant de la protéine TTP sur l'expression de l'ARNm du VEGF et de l'IL8 a été observé (Fig. 2B). Cette expérience confirme que la diminution des taux d'ARNm des facteurs angiogéniques dépend du taux de protéine TTP.

Pour évaluer la corrélation entre l'expression de la protéine TTP et la production de facteurs angiogéniques, les données obtenues pour les cellules MDA231 ont été confirmées en recourant à une approche complémentaire : l'inactivation (« silencing ») de l'expression de la protéine TTP dans la lignée de cellules mammaires cancéreuses MCF7, qui exprime normalement la protéine. Comme observé précédemment, les cellules MCF7 présentent une morphologie épithéliale et se caractérisent par des taux nettement détectables de la protéine TTP.

Deux clones exprimant deux sh-ARN indépendants (sh62.TTP et sh65.TTP) présentent une diminution de 30 % et 40 %, respectivement, des taux d'ARNm TTP, par rapport au contrôle (sh-ctrl) (Fig. 3A). Une analyse par immunoblot confirme une claire diminution des niveaux de TTP (Fig. 3B). Simultanément à la diminution de la protéine TTP, une augmentation des taux d'ARNm VEGF et IL8 a été observée dans les cellules sh62.TTP et sh65.TTP, ce qui confirme une corrélation inverse entre le niveau de TTP et les niveaux de VEGF et d'IL8 (Fig. 3C).

### II.3 L'effet de TTP sur la prolifération des cellules mammaires cancéreuses

Un autre phénotype tumorigène pouvant être modulé par l'expression de la protéine TTP est le taux de prolifération des cellules cancéreuses (Brennan *et al.,* 2009). Dans les deux clones MDA231 (PA1 et PA15), qui expriment la protéine TTP à des taux élevés et faibles, respectivement, une diminution du taux de prolifération dépend de l'expression de la protéine TTP (Fig. 4A). Lors de la prolifération des clones de MDA231 (jusqu'à quatre jours), aucun changement morphologique et aucune augmentation de mort cellulaire ou d'apoptose n'a été noté, comme observé pour le gliome ou les cellules HeLa (Suswam *et al.,* 2008, Brennan *et al.,* 2009). En outre, les clones » knock-down » MCF7-TTP stables (sh62-TTP1 et sh65-TTP2) ont été utilisés pour confirmer l'effet de l'expression de la protéine TTP sur la prolifération. D'un point de vue morphologique, ces clones ont la capacité de croître sous forme de colonies, un effet qui pourrait corroborer l'implication de la perte de la protéine TTP dans la transition épithéliale-mésenchymateuse (Gebeshuber *et al.,* 2009) (Fig. 4B). Les deux clones (sh62-TTP1 et sh65-TTP2) ont présenté une augmentation significative du taux de prolifération corrélée avec la disparition dose-dépendante de la protéine TTP (Fig. 4C). Comme décrit précédemment pour l'expression des ARNm du VEGF et de l'IL8, un effet dose-dépendant sur la prolifération a été observé.

### II.4 L'expression de TTP dans le cancer du sein

Considérées dans leur ensemble, les présentes données pointent clairement un rôle crucial des taux de protéine TTP dans la production de deux facteurs angiogéniques, le VEGF et l'IL8, et la régulation de la prolifération de cellules de cancer du sein. Ces résultats corroborent clairement les données des publications qui ont démontré la suppression fréquente de l'expression de la protéine TTP dans les cancers du sein. Par conséquent, la protéine TTP a été proposée en tant que facteur pronostic d'agressivité tumorale (Brennan *et al.,* 2009). La possibilité d'utiliser le taux d'expression de la protéine TTP comme facteur de pronostic du cancer du sein étant très prometteuse, la quantité relative d'ARNm de TTP et les niveaux de protéine TTP dans les lignées cellulaires mammaires cancéreuses décrites ci-dessus ont été testées afin de démontrer une corrélation entre les taux d'ARNm et de protéine TTP. Les cellules MCF7, MDA231, T47D et Hs578T ont été testées et les cellules embryonnaires de rein humain (HEK293) ont été utilisées en tant que contrôle négatif étant donné que ces cellules ont été décrites comme négatives pour l'expression de la protéine TTP (Lai *et al.,* 1999). Comme le montre la figure 5A, la protéine TTP peut être clairement détectée dans les cellules MCF7 et T47D alors qu'elle est absente ou très faiblement exprimée dans les cellules HEK293, MDA231 et Hs578T. L'ARNm de TTP a été analysé à l'aide de la méthode ΔΔCT Le taux d'ARNm dans les cellules MCF7 a été considéré comme valeur de référence (100 %). Les taux les plus faibles d'ARNm TTP ont été trouvés dans les cellules MDA231, tandis que des taux significatifs d'ARNm TTP ont été détectés dans les cellules MCF7 et T47D. Etonnamment, les cellules Hs578T qui n'expriment pas la protéine, ont des taux nettement détectables d'ARNm TTP (Fig. 5B). Ces données montrent une absence évidente de corrélation entre les taux d'expression d'ARNm et de protéine TTP. Cette absence de corrélation peut être due soit à une demi-vie plus courte de l'ARNm TTP ou à une différence des niveaux de traduction de son ARNm.

### II.5 Analyse génétique du gène TTP

Pour étudier l'absence de corrélation entre les taux de protéine et d'ARNm TTP dans les cellules Hs578T, la région codant la protéine TTP sur l'ADN génomique a été séquencée et l'ADN génomique des cellules MCF7 a été utilisé en tant que contrôle. Un polymorphisme mononucléotidique (SNP) a été détecté dans les cellules Hs578T (rs3746083=NM_003407.2: 367C>T, présence d'un nucléotide T au niveau du site polymorphe rs3746083) qui modifie le codon correspondant à l'arginine, transformant le CGC en CGT (R103R). Une étude cas-témoin a été réalisée pour évaluer le rôle joué par le variant 367C>T (présence d'un nucléotide T au niveau du site polymorphe rs3746083) dans la carcinogenèse mammaire en étudiant un groupe de femmes atteintes de cancer du sein (92 individus : groupe patientes) et un groupe de femmes contrôles (89 individus : groupe contrôles). Toutes les patientes présentaient des tumeurs très agressives portant une amplification du gène HER2 (HER2-positif) et tous les cas ont été traités à l'Herceptine® (trastuzumab), un anticorps monoclonal dirigé contre HER2 (Hall *et al.,* 2009). 13 hétérozygotes C/T au niveau du site polymorphe rs3746083 ont été identifiés dans le groupe des patientes (fréquence de l'allèle T=14,1 %) et 5 hétérozygotes C/T au niveau du site polymorphe rs3746083 ont été identifiés dans le groupe des contrôles (fréquence de l'allèle T=5,6 %). Les fréquences alléliques des allèles C et T du polymorphisme rs3746083 dans le tableau 1 ont révélé une augmentation de la fréquence de l'allèle T dans le groupe des patientes, n'atteignant cependant pas le seuil de significativité statistique (test p-Chi2=0,095 confirmé en utilisant le test p-Fisher=0,080, OR=2.7, CI95% [0,9-10,3]). En outre, il a été examiné si le variant 367C>T (présence d'un nucléotide T au niveau du site polymorphe rs3746083) pouvait être associé aux autres paramètres cliniques tels que le taux de survie. Aucune corrélation n'a été identifiée avec le taux de survie. Cependant, une distribution différente de l'allèle T du polymorphisme rs3746083 a été observée en ce qui concerne la réponse des patientes au traitement à l'Herceptine® (trastuzumab). Comme l'indique le tableau 2, l'allèle T du polymorphisme rs3746083 était nettement plus fréquent dans le groupe des patientes résistantes à l'Herceptine® (trastumuzab) que dans le groupe contrôle, avec une valeur statistique significative (test p-Chi2=0,010 confirmé par l'utilisation du test p-Fisher=0,0093, OR=8,0, CI95% [1,9-33,4]). Ces données indiquent que la présence d'un nucléotide T au niveau du site polymorphe rs3746083 pour au moins un allèle du gène codant la protéine TTP a un effet fonctionnel sur l'expression de la protéine TTP et est liée à une réaction différentielle des patients au traitement au trastuzumab (Herceptine®).

**Tableau 1. Distribution du variant C>T (présence d'un nucléotide T au niveau du site polymorphe rs3746083) chez 89 femmes contrôles et 92 patientes atteintes de cancer du sein HER2-positif.**

| Groupe | Hétérozygotie C/T au niveau du site polymorphe rs3746083 | Homozygotie C/C au niveau du site polymorphe rs3746083 | Total |
|---|---|---|---|
| Contrôles | 5 (5.6%) | 84(94.4%) | 89 (100%) |
| Patientes | 13 (14.1%) | 79(85.9%) | 92 (100%) |

**Tableau 2. Distribution du variant C>T (présence d'un nucléotide T au niveau du site polymorphe rs3746083) chez 54 patientes atteintes de cancer du sein et traitées avec l'Herceptine® (trastuzumab)**

| Non-répondeur | 6 (40%) | 9 (60%) | 15(100%) |
|---|---|---|---|
| Patientes | Hétérozygotie C/T au niveau du site polymorphe rs3746083 | Homozygotie C/C au niveau du site polymorphe rs3746083 | Total |
| Répondeur | 3(7.7%) | 36 (92.3%) | 39(100%) |

### II.6 Analyse fonctionnelle du variant génétique 367C>T (NM_003407.2) (présence d'un nucléotide T au niveau du site polymorphe rs3746083)

Considérant les résultats obtenus lors de l'analyse génétique, il a été examiné si le variant 367C>T (NM_003407.2) (présence d'un nucléotide T au niveau du site polymorphe rs3746083) pouvait être responsable de la divergence entre les taux d'ARNm et de protéine TTP observés dans les cellules Hs578T. Tout d'abord, il a été examiné si le changement 367C>T (NM_003407.2) pouvait affecter l'expression du gène en interférant avec la stabilité de l'ARNm ou la traduction de la protéine (Sauna, 2007). L'analyse par PCR de l'ADNc de TTP obtenue à partir de cellules Hs578T n'a pas révélé l'existence de transcrits alternatifs anormaux produits dans ces cellules en comparaison avec les cellules MDA231 (Fig. 6A). Afin de vérifier si tant le type sauvage (allèle C) (wt) que l'allèle variant (allèle T) (var) ont été transcrits, l'identification du nucléotide au niveau du site polymorphe rs3746083 a été réalisée d'une part par l'analyse de la digestion avec l'enzyme HhaI et d'autre part par le séquençage de produits de PCR issu d'ADN génomique et d'ADNc obtenus à partir de cellules Hs578T. Les deux techniques ont montré que l'allèle T (du polymorphisme rs3746083) était davantage exprimé que l'allèle C (du polymorphisme rs3746083) de type sauvage dans les cellules Hs578T (Fig. 6A et 6B). De plus, l'analyse de prédiction de la structure de l'ARNm TTP utilisant le programme MFold (http://rna.tbi.univie.ac.at/cgi-bin/RNAfold.cgi) a montré une structure secondaire plus stable des molécules d'ARNm correspondant à l'allèle T (du polymorphisme rs3746083) par rapport à celle des molécules d'ARNm correspondant à l'allèle C (du polymorphisme rs3746083) (Fig. 6C). La demi-vie de l'ARNm TTP est de 18 minutes dans les cellules MCF7 et MDA231 et de 21 minutes dans les cellules Hs578T (Fig. 6D). Comme les structures d'ARN hautement stables peuvent affecter le taux traductionnel de l'ARNm correspondant, il a été supposé que des molécules ARNm hautement stables et abondantes pouvaient être associées à des taux de protéine plus faibles (Nackley *et al.,* 2006). Pour tester fonctionnellement cette hypothèse, des expériences de transcription/traduction *in vitro* ont été réalisées à l'aide d'une construction TTP étiquetée tant pour l'allèle de type sauvage (allèle C du polymorphisme rs3746083) que pour l'allèle variant (allèle T du polymorphisme rs3746083). Ainsi, les taux de traduction du gène TTP de type sauvage (wt) (présence d'un nucléotide C au niveau du site polymorphe rs3746083) sont plus importants que ceux du gène TTP variant (var) (présence d'un nucléotide T au niveau du site polymorphe rs3746083) (Fig. 7A). Pour confirmer ces données, un modèle expérimental a été mis au point en clonant les deux régions codantes de pleine longueur (l'allèle C de type sauvage et l'allèle T variant du polymorphisme rs3746083) dans le vecteur d'expression eucaryote pcDNA4/TO/myc-His afin de procéder à une transfection transitoire dans la lignée cellulaire TTP-négative HEK293. Un gène rapporteur luciférase a été co-transfecté en tant que contrôle de l'efficacité de la transfection. Des expériences avec des quantités différentes de plasmides TTP (100, 200 et 500 ng d'ADN/10⁵ cellules) ont été réalisées pour déterminer la concentration optimale de TTP qui n'affectait pas la transcription et/ou la stabilité de la luciférase ou n'induisait pas une mort cellulaire massive. Aucun effet n'a été observé sur la mort cellulaire mais la transfection de 500 ng, tant de la construction de type sauvage (allèle C du polymorphisme rs3746083) que de celle du variant TTP (allèle T du polymorphisme rs3746083) a été exclue car elle affectait significativement l'activité de la luciférase. En ne comparant que les échantillons caractérisés par une efficacité de transfection comparable, nous montrons que le plasmide TTP de type sauvage (allèle C du polymorphisme rs3746083) produit une quantité plus importante de protéine que le plasmide TTP variant (allèle T du polymorphisme rs3746083) lorsque 100 ou 200 ng de plasmides d'expression sont transfectés (Fig. 7B).

Pour étudier davantage les différences entre les constructions de type sauvage (allèle C du polymorphisme rs3746083) et du variant TTP (allèle T du polymorphisme rs3746083), un dosage fonctionnel a été réalisé testant l'effet des deux plasmides sur les régions 3'UTR du VEGF, une cible de la protéine TTP bien connue, clonées en aval du gène rapporteur luciférase (Essafi-Benkhadir *et al.,* 2007). Notons que lorsque les cellules HEK293 ont été transfectées avec le gène TTP de type sauvage, une diminution de l'activité de la luciférase a été observée par rapport au contrôle (pcDNA4/TO/myc-His vecteur vide), mais la transfection de la forme variante de TTP donne une activité luciférase équivalente à celle obtenue après transfection du vecteur vide (Fig. 8A). Pour valider ces résultats, la demi-vie de l'ARNm VEGF endogène a été vérifiée dans les cellules HEK293. Une différence significative a été obtenue 24 heures après transfection des plasmides TTP de type sauvage. La transfection de la forme variante de TTP n'affecte pas de manière significative le temps de demi-vie de l'ARNm VEGF (Fig. 8B). Pour confirmer ces données, la transcription a été inhibée à l'aide de DRB et l'expression de cycline D1 a été vérifiée, l'ARNm de la cycline D1 étant une autre cible endogène de la protéine TTP (Marderosian *et al.,* 2006). Comme le montre la figure 8C, après 3 heures de traitement DRB, la quantité d'ARNm de cycline D1, a été diminuée seulement après transfection du gène TTP de type sauvage, la forme TTP variante n'ayant aucun effet. L'ensemble de ces données montrent que les formes d'ARNm TTP correspondant à l'allèle T du polymorphisme rs3746083 (présence d'un nucléotide T au niveau du site polymorphe rs3746083) n'ont pas été traduites avec la même efficacité *in vitro* et *in vivo* résultant en des taux inférieurs de protéine. La présence d'ARNm TTP correspondant à l'allèle T du polymorphisme rs3746083 (présence d'un nucléotide T au niveau du site polymorphe rs3746083) se manifeste par des taux inférieurs de protéine résultant en une augmentation de la demi-vie d'ARNm cibles.

### REFERENCES BIBLIOGRAPHIQUES

Al-Souhibani, N., Al-Ahmadi, W., Hesketh, J.E., Blackshear, P.J., Khabar, K.S. (2010) The RNA-binding zinc-finger protein tristetraprolin regulates AU-rich mRNAs involved in breast cancer-related processes. Oncogene., 29, 4205-4215.
Bargmann CI, Hung MC, Weinberg RA.. 1986. The neu oncogene encodes an epidermal growth factor receptor-related protein. Nature Jan 16-22;319(6050):226-30
Brennan, S.E., Kuwano, Y., Alkharouf, N., Blackshear, P.J., Gorospe, M., Wilson, G.M. (2009) The mRNA-destabilizing protein tristetraprolin is suppressed in many cancers, altering tumorigenic phenotypes and patient prognosis. Cancer Res., 69, 5168-5176.
Chen JS, Lan K, Hung MC. 2003 Stratégies to target HER2/neu overexpression for cancer therapy.Drug Resist Updat. Jun;6(3): 129-36. Review. Erratum in: Drug Resist Updat. 2003 Oct;6(5):296.
De Laurentiis, M., Cancello, G., Zinno, L., Montagna, E., Malorni, L., Esposito, A., Pennacchio, R., Silvestro, L., Giuliano, M., Giordano, A., et al. (2005). Targeting HER2 as a therapeutic strategy for breast cancer: a paradigmatic shift of drug development in oncology. Ann Oncol 16 Suppl 4, iv7-iv13.
Eckert, L.B., Repasky, G.A., Ulkü, A.S., McFall, A., Zhou, H., Sartor, C.I., Der, C.J. (2004) Involvement of Ras activation in human breast cancer cell signaling, invasion, and anoikis. Cancer Res., 64, 4585-4592.
Essafi-Benkhadir, K., Onesto, C., Stebe, E., Moroni, C., Pages, G. (2007) Tristetraprolin inhibits Ras-dependent Tumor vascularization by inducing Vascular Endothelial Growth factor mRNA degradation. Mol. Biol. Cell., 18, 4648-4658.
Gebeshuber, C.A., Zatloukal, K., Martinez, J. (2009) miR-29a suppresses tristetraprolin, which is a regulator of epithelial polarity and metastasis. EMBO Rep., 10, 400-405.
Gianni L, Dafni U, Gelber RD, Azambuja E, Muehlbauer S, Goldhirsch A, Untch M, Smith I, Baselga J, Jackisch C, Cameron D, Mano M, Pedrini JL, Veronesi A, Mendiola C, Pluzanska A, Semiglazov V, Vrdoljak E, Eckart MJ, Shen Z, Skiadopoulos G, Procter M, Pritchard KI, Piccart-Gebhart MJ, Bell R; Herceptin Adjuvant (HERA) Trial Study Team. 2011 Treatment with trastuzumab for 1 year after adjuvant chemotherapy in patients with HER2-positive early breast cancer: a 4-year follow-up of a randomised controlled trial. Lancet Oncol. Mar;12(3):236-44. Epub 2011 Feb 25.
Hall, P.S., Cameron, D.A. (2009) Current perspective - trastuzumab. Eur. J. Cancer, 45,12-18.
Johnson, B.A., Geha, M., Blackwell, T.K. (2000) Similar but distinct effects of the tristetraprolin/TIS11 immediate-early proteins on cell survival. Oncogene, 19, 1657-1664.
Lai, W.S., Carballo, E., Strum, J.R., Kennington, E.A., Phillips, R.S., Blackshear, P.J. (1999) Evidence that tristetraprolin binds to AU-rich elements and promotes the deadenylation and destabilization of tumor necrosis factor alpha mRNA. Mol. Cell. Biol., 19, 4311-4323.
Marderosian, M., Sharma, A., Funk, A.P., Vartanian, R., Masri, J., Jo, O.D., Gera, J.F. (2006) Tristetraprolin regulates Cyclin D1 and c-Myc mRNA stability in response to rapamycin in an Akt-dependent manner via p38 MAPK signaling. Oncogene, 25, 6277-6290.
Nackley, A.G., Shabalina, S.A., Tchivileva, I.E., Satterfield, K., Korchynskyi, O., Makarov, S.S., Maixner, W., Diatchenko, L. (2006) Human catechol-O-methyltransferase haplotypes modulate protein expression by altering mRNA secondary structure. Science, 314, 1930-1933.
Nahta R, Esteva FJ. 2006 HER2 therapy: molecular mechanisms of trastuzumab resistance. Breast Cancer Res.;8(6):215.
Sauna, Z.E., Kimchi-Sarfaty, C., Ambudkar, S.V., Gottesman, M.M. (2007) Silent polymorphisms speak: how they affect pharmacogenomics and the treatment of cancer. Cancer Res., 67, 9609-9612.
Scatchard, G. 1949. The attractions of proteins for small molecules and ions. Ann. N.Y. Acad. Sci. 51 660-672
Schmittgen, T.D., Livak, K.J. (2008) Analyzing real-time PCR data by the comparative C(T) method. Nat. Protoc., 3, 1101-1108.
Slamon, D. J., Clark, G. M., Wong, S. G., Levin, W. J., Ullrich, A., and McGuire, W. L. (1987). Human breast cancer: correlation of relapse and survival with amplification of the HER-2/neu oncogene. Science 235, 177-182.
Suswam, E., Li, Y., Zhang, X., Gillespie, G.Y, Li, X., Shacka, J.J., Lu, L., Zheng, L., King, P.H. (2008) Tristetraprolin down-regulates interleukin-8 and vascular endothelial growth factor in malignant glioma cells. Cancer Res., 68, 674-682.
Yamamoto T, Ikawa S, Akiyama T, Semba K, Nomura N, Miyajima N, Saito T, Toyoshima K. 1986 Similarity of protein encoded by the human c-erb-B-2 gene to epidermal growth factor receptor. Nature. Jan 16-22;319(6050):230-4.

## Revendications

1. Méthode *in vitro* ou *ex vivo* de prédiction de la réponse d'un sujet à un traitement avec au moins un agent bloquant HER2, ladite méthode comprenant les étapes de :
i) identification du nucléotide au niveau du site polymorphe rs3746083 367C>T sur la séquence SEQ ID NO : 3, pour au moins un allèle, en particulier les deux allèles du gène codant la protéine tristétraproline, dans un échantillon biologique dudit sujet ; et/ou
ii) détermination du taux de protéine tristétraproline, dans un échantillon biologique dudit sujet ;
dans laquelle ledit sujet est atteint d'un cancer du sein HER2-positif ; et
dans laquelle ledit agent bloquant est le trastuzumab.

2. Méthode selon la revendication 1, comprenant en outre l'étape de :
iii) comparaison du taux de protéine tristétraproline dans un échantillon biologique dudit sujet déterminé à l'étape ii) avec au moins une valeur référence.

3. Agent bloquant HER2, pour son utilisation en tant que médicament pour le traitement d'un cancer du sein HER2-positif chez un sujet, ledit sujet étant prédit comme répondeur à un traitement avec ledit agent bloquant HER2 par la méthode telle que définie selon l'une des revendications 1 ou 2, ledit agent bloquant étant le trastuzumab.

4. Produit comprenant :
- au moins un agent bloquant HER2 ; et
- au moins un autre agent de traitement d'un cancer HER2-positif,
en tant que produit de combinaison pour une utilisation simultanée, séparée ou étalée dans le temps pour le traitement d'un cancer du sein HER2-positif chez un sujet, ledit sujet étant prédit comme répondeur à un traitement avec ledit agent bloquant HER2 par la méthode telle que définie selon l'une des revendications 1 ou 2, ledit agent bloquant étant le trastuzumab

5. Méthode *in vitro* ou *ex vivo* de pronostic ou de diagnostic d'un cancer chez un sujet comprenant :
α) l'identification du nucléotide au niveau du site polymorphe rs3746083 367C>T sur la séquence SEQ ID NO : 3, pour au moins un allèle, en particulier les deux allèles du gène codant la protéine tristétraproline dans un échantillon biologique dudit sujet,
dans laquelle le cancer est un cancer du sein HER2-positif de mauvais pronostic.

## Patentansprüche

1. *In vitro-* oder *ex vivo*-Verfahren zur Vorhersage der Reaktion eines Subjekts auf eine Behandlung mit mindestens einem HER2-Blockierungsmittel, wobei das Verfahren die folgenden Schritte umfasst:
i) Identifizieren des Nukleotids auf dem Niveau der polymorphen Stelle rs3746083 367C>T auf der Sequenz SEQ ID Nr.: 3 für mindestens ein Allel, insbesondere die zwei Allele des Gens, das das Protein Tristetraprolin codiert, in einer biologischen Probe des Subjekts; und/oder
ii) Bestimmen der Rate des Proteins Tristetraprolin in einer biologischen Probe des Subjekts;
wobei das Subjekt an einem HER2-positiven Brustkrebs leidet; und
wobei das Blockierungsmittel Trastuzumab ist.

2. Verfahren nach Anspruch 1, umfassend außerdem den Folgenden Schritt:
iii) Vergleichen der Rate des Proteins Tristetraprolin in einer biologischen Probe des Subjekts, bestimmt in Schritt ii), mit mindestens einem Referenzwert.

3. HER2-Blockierungsmittel für seine Verwendung als Medikament für die Behandlung eines HER2-positiven Brustkrebses bei einem Subjekt, wobei vorhergesagt wurde, dass das Subjekt auf eine Behandlung mit dem HER2-Blockierungsmittel reagiert, durch das Verfahren, wie nach einem der Ansprüche 1 oder 2 definiert, wobei das Blockierungsmittel Trastuzumab ist.

4. Produkt, umfassend:
- mindestens ein HER2-Blockierungsmittel; und
- mindestens ein weiteres Mittel zur Behandlung eines HER2-positiven Krebses,
als Kombinationsprodukt für eine gleichzeitige, getrennte oder zeitlich gestaffelte Verwendung für die Behandlung eines HER2-positiven Brustkrebses bei einem Subjekt, wobei vorhergesagt wurde, dass das Subjekt auf eine Behandlung mit dem HER2-Blockierungsmittel reagiert, durch das Verfahren, wie nach einem der Ansprüche 1 oder 2 definiert, wobei das Blockierungsmittel Trastuzumab ist.

5. *In vitro-* oder *ex vivo*-Verfahren zur Prognose oder zur Diagnose eines Krebses bei einem Subjekt, umfassend:
a) Identifizieren des Nukleotids auf dem Niveau der polymorphen Stelle rs3746083 367C>T auf der Sequenz SEQ ID Nr.: 3 für mindestens ein Allel, insbesondere die zwei Allele des Gens, das das Protein Tristetraprolin codiert, in einer biologischen Probe des Subjekts,
wobei der Krebs ein HER2-positiver Brustkrebs mit schlechter Prognose ist.

## Claims

1. An in vitro or ex vivo method for predicting the response of a patient to treatment with at least one HER2-blocking agent, said method including the steps of:
i) identifying the nucleotide at the rs3746083 367C>T polymorphic site of sequence SEQ ID NO : 3, for at least one allele, in particular both alleles of the gene coding for the tristetraprolin protein in a biological sample from said patient; and/or
ii) determining the level of tristetraprolin protein in a biological sample from said patient;
wherein said patient is suffering from HER2-positive breast cancer, and
wherein said blocking agent is trastuzumab.

2. The method according to claim 1, further including the step of:
iii) comparing the level of tristetraprolin protein in a biological sample from said patient determined in step ii) with at least one reference value.

3. An HER2-blocking agent for use as a drug for treating HER2-positive breast cancer in a patient, said patient being predicted to be responsive to treatment with said HER2-blocking agent by the method as defined in any one of claims 1 or 2, said blocking agent being trastuzumab.

4. A product including:
- at least one HER2-blocking agent; and
- at least one other agent for treating HER2-positive cancer,
as a combination product for simultaneous, separate or sequential use for treating HER2-positive breast cancer in a patient, said patient being predicted to be responsive to treatment with said HER2-blocking agent by the method as defined according to any one of claims 1 or 2, said blocking agent being trastuzumab.

5. An in vitro or ex vivo method of prognosis or diagnosis of cancer in a patient including:
a) identifying the nucleotide at the rs3746083 367C>T polymorphic site of sequence SEQ ID NO : 3, for at least one allele, in particular both alleles of the gene coding for the tristetraprolin protein in a biological sample from said patient,
wherein the cancer is HER2-positive breast cancer with a poor prognosis.


### SEQUENCE LISTING

<110> CENTRE NATIONAL DE LA RECHERCHE SCIENTIFIQUE (CNRS) UNIVERSITE DE NICE SOPHIA ANTIPOLIS
<120> METHODE DE PREDICTION DE LA REPONSE A UN TRAITEMENT AVEC UN AGENT BLOQUANT HER2
<130> 360965D29632
<150> FR 1155128
   <151> 2011-06-10
<160> 7
<170> PatentIn version 3.5
<210> 1
   <211> 1255
   <212> PRT
   <213> homo sapiens
<400> 1
<210> 2
   <211> 326
   <212> PRT
   <213> homo sapiens
<400> 2
   Met Asp Leu Thr Ala Ile Tyr Glu Ser Leu Leu Ser Leu Ser Pro Asp
   1               5                   10                  15
   Val Pro Val Pro Ser Asp His Gly Gly Thr Glu Ser Ser Pro Gly Trp
               20                  25                  30
   Gly Ser Ser Gly Pro Trp Ser Leu Ser Pro Ser Asp Ser Ser Pro Ser
           35                  40                  45
   Gly Val Thr Ser Arg Leu Pro Gly Arg Ser Thr Ser Leu Val Glu Gly
       50                  55                  60
   Arg Ser Cys Gly Trp Val Pro Pro Pro Pro Gly Phe Ala Pro Leu Ala
   65                  70                  75                  80
   Pro Arg Leu Gly Pro Glu Leu Ser Pro Ser Pro Thr Ser Pro Thr Ala
                   85                  90                  95
   Thr Ser Thr Thr Pro Ser Arg Tyr Lys Thr Glu Leu Cys Arg Thr Phe
               100                 105                 110
   Ser Glu Ser Gly Arg Cys Arg Tyr Gly Ala Lys Cys Gln Phe Ala His
           115                 120                 125
   Gly Leu Gly Glu Leu Arg Gln Ala Asn Arg His Pro Lys Tyr Lys Thr
       130                 135                 140
   Glu Leu Cys His Lys Phe Tyr Leu Gln Gly Arg Cys Pro Tyr Gly Ser
   145                 150                 155                 160
   Arg Cys His Phe Ile His Asn Pro Ser Glu Asp Leu Ala Ala Pro Gly
                   165                 170                 175
   His Pro Pro Val Leu Arg Gln Ser Ile Ser Phe Ser Gly Leu Pro Ser
               180                 185                 190
   Gly Arg Arg Thr Ser Pro Pro Pro Pro Gly Leu Ala Gly Pro Ser Leu
           195                 200                 205
   Ser Ser Ser Ser Phe Ser Pro Ser Ser Ser Pro Pro Pro Pro Gly Asp
       210                 215                 220
   Leu Pro Leu Ser Pro Ser Ala Phe Ser Ala Ala Pro Gly Thr Pro Leu
   225                 230                 235                 240
   Ala Arg Arg Asp Pro Thr Pro Val Cys Cys Pro Ser Cys Arg Arg Ala
                   245                 250                 255
   Thr Pro Ile Ser Val Trp Gly Pro Leu Gly Gly Leu Val Arg Thr Pro
               260                 265                 270
   Ser Val Gln Ser Leu Gly Ser Asp Pro Asp Glu Tyr Ala Ser Ser Gly
           275                 280                 285
   Ser Ser Leu Gly Gly Ser Asp Ser Pro Val Phe Glu Ala Gly Val Phe
       290                 295                 300
   Ala Pro Pro Gln Pro Val Ala Ala Pro Arg Arg Leu Pro Ile Phe Asn
   305                 310                 315                 320
   Arg Ile Ser Val Ser Glu
                   325
<210> 3
   <211> 1745
   <212> DNA
   <213> Homo sapiens
<400> 3
   agcctgactt cagcgctccc actctcggcc gacacccctc atggccaacc gttacaccat 60
   ggatctgact gccatctacg agagcctcct gtcgctgagc cctgacgtgc ccgtgccatc 120
   cgaccatgga gggactgagt ccagcccagg ctggggctcc tcgggaccct ggagcctgag 180
   cccctccgac tccagcccgt ctggggtcac ctcccgcctg cctggccgct ccaccagcct 240
   agtggagggc cgcagctgtg gctgggtgcc cccaccccct ggcttcgcac cgctggctcc 300
   ccgcctgggc cctgagctgt caccctcacc cacttcgccc actgcaacct ccaccacccc 360
   ctcgcgctac aagactgagc tatgtcggac cttctcagag agtgggcgct gccgctacgg 420
   ggccaagtgc cagtttgccc atggcctggg cgagctgcgc caggccaatc gccaccccaa 480
   atacaagacg gaactctgtc acaagttcta cctccagggc cgctgcccct acggctctcg 540
   ctgccacttc atccacaacc ctagcgaaga cctggcggcc ccgggccacc ctcctgtgct 600
   tcgccagagc atcagcttct ccggcctgcc ctctggccgc cggacctcac caccaccacc 660
   aggcctggcc ggcccttccc tgtcctccag ctccttctcg ccctccagct ccccaccacc 720
   acctggggac cttccactgt caccctctgc cttctctgct gcccctggca cccccctggc 780
   tcgaagagac cccaccccag tctgttgccc ctcctgccga agggccactc ctatcagcgt 840
   ctgggggccc ttgggtggcc tggttcggac cccctctgta cagtccctgg gatccgaccc 900
   tgatgaatat gccagcagcg gcagcagcct ggggggctct gactctcccg tcttcgaggc 960
   gggagttttt gcaccacccc agcccgtggc agccccccgg cgactcccca tcttcaatcg 1020
   catctctgtt tctgagtgac aaagtgactg cccggtcaga tcagctggat ctcagcgggg 1080
   agccacgtct cttgcactgt ggtctctgca tggaccccag ggctgtgggg acttggggga 1140
   cagtaatcaa gtaatcccct tttccagaat gcattaaccc actcccctga cctcacgctg 1200
   gggcaggtcc ccaagtgtgc aagctcagta ttcatgatgg tgggggatgg agtgtcttcc 1260
   gaggttcttg ggggaaaaaa aattgtagca tatttaaggg aggcaatgaa ccctctcccc 1320
   cacctcttcc ctgcccaaat ctgtctccta gaatcttatg tgctgtgaat aataggcctt 1380
   cactgcccct ccagttttta tagacctgag gttccagtgt ctcctggtaa ctggaacctc 1440
   tcctgagggg gaatcctggt gctcaaatta ccctccaaaa gcaagtagcc aaagccgttg 1500
   ccaaacccca cccataaatc aatgggccct ttatttatga cgactttatt tattctaata 1560
   tgattttata gtatttatat atattgggtc gtctgcttcc cttgtatttt tcttcctttt 1620
   tttgtaatat tgaaaacgac gatataatta ttataagtag actataatat atttagtaat 1680
   atatattatt accttaaaag tctatttttg tgttttgggc atttttaaat aaacaatctg 1740
   agtgt 1745
<210> 4
   <211> 20
   <212> DNA
   <213> artificial
<220>
   <223> amorce sens
<400> 4
   ccactctcgg ccgacacccc 20
<210> 5
   <211> 22
   <212> DNA
   <213> artificial
<220>
   <223> amorce antisens
<400> 5
   gtcactcaga aacagagatg cg 22
<210> 6
   <211> 19
   <212> DNA
   <213> artificial
<220>
   <223> amorce 103F
<400> 6
   cgaccatgga gggactgag 19
<210> 7
   <211> 20
   <212> DNA
   <213> artificial
<220>
   <223> amorce 103R
<400> 7
   gccctggagg tagaacttgt 20